(19) Europäisches Patentamt
European Patent Office
Office européen des brevets

(11) EP 4 369 347 B1

(12) EUROPEAN PATENT SPECIFICATION

(45) Date of publication and mention of the grant of the patent:
**30.04.2025 Bulletin 2025/18**

(21) Application number: **23200931.6**

(22) Date of filing: **29.09.2023**

(51) International Patent Classification (IPC):
***G16C 60/00*** *(2019.01)* ***C09D 1/00*** *(2006.01)*
***C09D 175/04*** *(2006.01)* *G16C 20/30* *(2019.01)*

(52) Cooperative Patent Classification (CPC):
**G16C 60/00; C09D 175/04;** G16C 20/30

# (54) SYSTEMS AND METHODS FOR PREDICTING PROPERTIES OF COATING TYPES AND ESTIMATING SERVICE LIFE THEREOF

SYSTEME UND VERFAHREN ZUR VORHERSAGE DER EIGENSCHAFTEN VON BESCHICHTUNGSARTEN UND SCHÄTZUNG DER LEBENSDAUER DAVON

SYSTÈMES ET PROCÉDÉS DE PRÉDICTION DE PROPRIÉTÉS DE TYPES DE REVÊTEMENT ET D'ESTIMATION DE DURÉE DE VIE DE CEUX-CI

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **10.11.2022 IN 202221064312**

(43) Date of publication of application:
**15.05.2024 Bulletin 2024/20**

(73) Proprietor: **Tata Consultancy Services Limited**
**Maharashtra (IN)**

(72) Inventors:
- **SHRIVASTAVA, SURABHI**
  **411013 Pune - Maharashtra (IN)**
- **KAUSLEY, SHANKAR BALAJIRAO**
  **411013 Pune -, Maharashtra (IN)**
- **BHATTACHARJEE, SURYADIP**
  **411013 Pune - Maharashtra (IN)**
- **MAITI, SOUMYADIPTA**
  **411013 Pune - Maharashtra (IN)**
- **RAI, BEENA**
  **411013 Pune - Maharashtra (IN)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
- **CROLL S. ET AL: "A framework for predicting the service lifetime of composite polymeric coatings", JOURNAL OF MATERIAL SCIENCE, KLUWER ACADEMIC PUBLISHERS, DORDRECHT, vol. 43, no. 20, 1 October 2008 (2008-10-01), pages 6630 - 6641, XP036678744, ISSN: 0022-2461, [retrieved on 20081001], DOI: 10.1007/S10853-008-2645-7**
- **CROLL S. G.: "Application and limitations of current understanding to model failure modes in coatings", JOURNAL OF COATINGS TECHNOLOGY AND RESEARCH, SPRINGER US, BOSTON, vol. 10, no. 1, 27 October 2012 (2012-10-27), pages 15 - 27, XP035165909, ISSN: 1935-3804, DOI: 10.1007/S11998-012-9443-5**
- **CROLL S. G. ET AL: "Statistical approaches for predicting weathering degradation and service life", PROCESS IN ORGANIC COATINGS, ELSEVIER BV, NL, vol. 55, no. 2, 1 February 2006 (2006-02-01), pages 75 - 87, XP027906424, ISSN: 0300-9440, [retrieved on 20060201]**
- **GUSEVA O. ET AL: "Service life prediction for aircraft coatings", POLYMER DEGRADATION AND STABILITY, BARKING, GB, vol. 82, no. 1, 1 January 2003 (2003-01-01), pages 1 - 13, XP004450525, ISSN: 0141-3910, DOI: 10.1016/S0141-3910(03)00124-1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 369 347 B1

- KIIL S.: "Model-based analysis of photoinitiated coating degradation under artificial exposure conditions", JOURNAL OF COATINGS TECHNOLOGY AND RESEARCH, SPRINGER US, BOSTON, vol. 9, no. 4, 4 January 2012 (2012-01-04), pages 375 - 398, XP035071707, ISSN: 1935-3804, DOI: 10.1007/S11998-011-9383-5

## Description

CROSS-REFERENCE TO RELATED APPLICATIONS AND PRIORITY

The present application claims priority to Indian application no. 202221064312, filed on November 10, 2022.

TECHNICAL FIELD

The disclosure herein generally relates to simulating techniques, and, more particularly, to systems and methods for predicting properties of coating types and estimating service life thereof.

BACKGROUND

Coating materials (often known as coatings) such as paints offer dual benefits of aesthetics and protection of a substrate from the outside damaging elements. A key part of coating formulation is to predict and maximize its service life. Currently, coating development and testing is a very costly and time-consuming process in the industry. Usually, a newly developed coating is kept under natural sunshine for more than 5 years to examine the gradual effects of weathering. In a life cycle of coating materials, it is subjected to varied dynamic weathering conditions like, light, humidity, acidic environment, chemical impacts, etc. wherein destructive experimental testing is performed and these testing are accelerated to speed up the overall results. However, accelerated testing setup does not capture real effects. Further, there are difficulties in gathering inputs for model development such as material properties, system parameters, and the like. Such challenges result in obtain inaccurate results thereby leading to lack in comprehensive models. Croll S. et al. (2008) "A framework for predicting the service lifetime of composite polymeric coatings", JOURNAL OF MATERIAL SCIENCE, vol. 43, no. 20, pages 6630-6641 discloses an approach for modeling degradation in polymeric coatings, which may be applicable to other systems when they are exposed to natural or artificial weathering conditions. Croll S. G. (2012) "Application and limitations of current understanding to model failure modes in coatings", JOURNAL OF COATINGS TECHNOLOGY AND RESEARCH, vol. 10, no. 1, pages 15-27 is a review on stochastic models used to predict coating properties, such as gloss, toughness, corrosion protection, etc. Croll S. G. et al. (2006) "Statistical approaches for predicting weathering degradation and service life", PROCESS IN ORGANIC COATINGS, vol. 55, no. 2, pages 75-87 is a review on statistical methods for predicting weathering degradation of materials, in particular pigmented coatings, and how it influences their service life. Guseva O. et al. (2003) "Service life prediction for aircraft coatings", POLYMER DEGRADATION AND STABILITY, vol. 82, no. 1, pages 1-13 discloses a model with three stress types, the temperature, UV and aerosol, for estimating the service life of organic coatings under service conditions. Kiil S. (2012) "Model-based analysis of photoinitiated coating degradation under artificial exposure conditions", JOURNAL OF COATINGS TECHNOLOGY AND RESEARCH, vol. 9, no. 4, pages 375-398 investigates coating degradation mechanisms of thermoset coatings exposed to ultraviolet radiation and humidity at constant temperature by using a transient 1-D mathematical model.

SUMMARY

Embodiments of the present disclosure present technological improvements as solutions to one or more of the above-mentioned technical problems recognized by the inventors in conventional systems.

For example, in one aspect, there is provided a processor implemented method for predicting properties of coating types and estimating service life thereof. The method comprises obtaining, via one or more hardware processors, a coating type, and one or more weathering conditions that the coating type is to be subjected in an environment; identifying a polymer for the one or more weathering conditions for the coating type; identifying one or more relevant reactions based on the identified polymer and the one or more weathering conditions; estimating a mass balance equation for each chemical component comprised in the one or more relevant reactions; and solving the mass balance equation for each chemical component to (i) predict one or more properties of the coating type, and (ii) obtain an estimated service life.

In an embodiment, the step of solving the mass balance equation for each chemical component comprises: processing the mass balance equation for each chemical component by a first Partial Difference Equations (PDE) solver to obtain (i) a first concentration profile, and (ii) a second concentration profile comprising an associated depth for each chemical component; estimating, by using a first chemical-physical correlator, a first set of chemical-physical correlations of constituents comprised in the coating type based on (i) the second concentration profile comprising the associated depth and (ii) time at which the coating type is applied and (iii) the one or more weathering conditions that the coating type is to be subjected in the environment; estimating, via an optimizer, (i) the second concentration profile comprising the associated depth and (ii) the time at which the coating type is applied and (iii) the one or more weathering conditions that the coating type is to be subjected in the environment to one or more optimized reactions-based parameters; processing the one or more optimized reactions-based parameters via a second PDE solver and a third PDE solver to obtain a third

concentration profile, and a fourth concentration profile, respectively; and estimating, by using a second chemical-physical correlator, a second set of chemical-physical correlations of constituents comprised in the coating type using at least one of the third concentration profile, and the fourth concentration profile.

In an embodiment, the method further comprises determining a change in surface roughness of the coating type with reference to time based on the second set of chemical-physical correlations of constituents comprised in the coating type.

In an embodiment, the method further comprises determining a change in thickness of the coating type with reference to time based on the first set of chemical-physical correlations and the second set of chemical-physical correlations of constituents comprised in the coating type.

In an embodiment, the method further comprises determining a change in (i) gloss, (ii) a Griffith's stress, and (iii) a wetting angle of the coating type based on the change in surface roughness of the coating type with reference to time.

In an embodiment, the estimated service life is obtained based on at least one of (i) the change in surface roughness of the coating type, (ii) the change in thickness of the coating type, (iii) the gloss, (iv) the Griffith's stress, and (v) the wetting angle of the coating type.

In another aspect, there is provided a processor implemented system for predicting properties of coating types and estimating service life thereof. The system comprises: a memory storing instructions; one or more communication interfaces; and one or more hardware processors coupled to the memory via the one or more communication interfaces, wherein the one or more hardware processors are configured by the instructions to: obtain a coating type, and one or more weathering conditions that the coating type is to be subjected in an environment; identify a polymer for the one or more weathering conditions for the coating type; identify one or more relevant reactions based on the identified polymer and the one or more weathering conditions; estimate a mass balance equation for each chemical component comprised in the one or more relevant reactions; and solve the mass balance equation for each chemical component to (i) predict one or more properties of the coating type, and (ii) obtain an estimated service life.

In an embodiment, the mass balance equation for each chemical component is solved by: processing the mass balance equation for each chemical component by a first Partial Difference Equations (PDE) solver to obtain (i) a first concentration profile, and (ii) a second concentration profile comprising an associated depth for each chemical component; estimating, by using a first chemical-physical correlator, a first set of chemical-physical correlations of constituents comprised in the coating type based on (i) the second concentration profile comprising the associated depth and (ii) time at which the coating type is applied and (iii) the one or more weathering conditions that the coating type is to be subjected in the environment; estimating, via an optimizer, (i) the second concentration profile comprising the associated depth and (ii) the time at which the coating type is applied and (iii) the one or more weathering conditions that the coating type is to be subjected in the environment to one or more optimized reactions-based parameters; processing the one or more optimized reactions-based parameters via a second PDE solver and a third PDE solver to obtain a third concentration profile, and a fourth concentration profile, respectively; and estimating, by using a second chemical-physical correlator, a second set of chemical-physical correlations of constituents comprised in the coating type using at least one of the third concentration profile, and the fourth concentration profile.

In an embodiment, the one or more hardware processors are further configured by the instructions to determine a change in surface roughness of the coating type with reference to time based on the second set of chemical-physical correlations of constituents comprised in the coating type.

In an embodiment, the one or more hardware processors are further configured by the instructions to determine a change in thickness of the coating type with reference to time based on the first set of chemical-physical correlations and the second set of chemical-physical correlations of constituents comprised in the coating type.

In an embodiment, the one or more hardware processors are further configured by the instructions to determine a change in (i) gloss, (ii) a Griffith's stress, and (iii) a wetting angle of the coating type based on the change in surface roughness of the coating type with reference to time.

In an embodiment, the estimated service life is obtained based on at least one of (i) the change in surface roughness of the coating type, (ii) the change in thickness of the coating type, (iii) the gloss, (iv) the Griffith's stress, and (v) the wetting angle of the coating type.

In yet another aspect, there are provided one or more non-transitory machine-readable information storage mediums comprising one or more instructions which when executed by one or more hardware processors cause predicting properties of coating types and estimating service life thereof by: obtaining a coating type, and one or more weathering conditions that the coating type is to be subjected in an environment; identifying a polymer for the one or more weathering conditions for the coating type; identifying one or more relevant reactions based on the identified polymer and the one or more weathering conditions; estimating a mass balance equation for each chemical component comprised in the one or more relevant reactions; and solving the mass balance equation for each chemical component to (i) predict one or more properties of the coating type, and (ii) obtain an estimated service life.

In an embodiment, the step of solving the mass balance equation for each chemical component comprises: processing the mass balance equation for each chemical component by a first Partial Difference Equations (PDE) solver to

obtain (i) a first concentration profile, and (ii) a second concentration profile comprising an associated depth for each chemical component; estimating, by using a first chemical-physical correlator, a first set of chemical-physical correlations of constituents comprised in the coating type based on (i) the second concentration profile comprising the associated depth and (ii) time at which the coating type is applied and (iii) the one or more weathering conditions that the coating type is to be subjected in the environment; estimating, via an optimizer, (i) the second concentration profile comprising the associated depth and (ii) the time at which the coating type is applied and (iii) the one or more weathering conditions that the coating type is to be subjected in the environment to one or more optimized reactions-based parameters; processing the one or more optimized reactions-based parameters via a second PDE solver and a third PDE solver to obtain a third concentration profile, and a fourth concentration profile, respectively; and estimating, by using a second chemical-physical correlator, a second set of chemical-physical correlations of constituents comprised in the coating type using at least one of the third concentration profile, and the fourth concentration profile.

In an embodiment, the method further comprises determining a change in surface roughness of the coating type with reference to time based on the second set of chemical-physical correlations of constituents comprised in the coating type.

In an embodiment, the method further comprises determining a change in thickness of the coating type with reference to time based on the first set of chemical-physical correlations and the second set of chemical-physical correlations of constituents comprised in the coating type.

In an embodiment, the method further comprises determining a change in (i) gloss, (ii) a Griffith's stress, and (iii) a wetting angle of the coating type based on the change in surface roughness of the coating type with reference to time.

In an embodiment, the estimated service life is obtained based on at least one of (i) the change in surface roughness of the coating type, (ii) the change in thickness of the coating type, (iii) the gloss, (iv) the Griffith's stress, and (v) the wetting angle of the coating type.

It is to be understood that both the foregoing general description and the following detailed description are exemplary and explanatory only and are not restrictive of the invention, as claimed.

## BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 depicts an exemplary system for predicting properties of coating types and estimating service life thereof, in accordance with an embodiment of the present disclosure.

FIG. 2 depicts an exemplary high level flow diagram of the system for predicting properties of coating types and estimating service life thereof, in accordance with an embodiment of the present disclosure.

FIG. 3 depicts an exemplary high level flow diagram of the system 100 for predicting properties of coating types and estimating service life thereof, in accordance with an embodiment of the present disclosure.

FIG. 4 depicts an exemplary flow chart illustrating a method for predicting properties of coating types and estimating service life thereof, using the system of FIG. 1, and the exemplary flow diagrams of the FIGS. 2-3, in accordance with an embodiment of the present disclosure.

FIGS. 5A through 5C depict a graphical representation illustrating a comparison of mean-squared error (MSE) estimated (i) during simulation by the system 100 and (ii) by conventional research work (Larche et al.), in accordance with an embodiment of the present disclosure.

FIGS. 6A through 6D depict a graphical representation illustrating a concentration profile of main polyurethane, in accordance with an embodiment of the present disclosure.

FIG. 7 depicts a graphical representation illustrating thickness reduction with time from 1-D space for the coating type, in accordance with an embodiment of the present disclosure.

FIG. 8 depicts a schematic of 2D simulations performed by the system of FIG. 1, in accordance with an embodiment of the present disclosure.

FIGS. 9A and 9B depict length of coating in $\mu$m, in accordance with an embodiment of the present disclosure.

FIGS. 10A-10B depict a graphical representation illustrating a normalized concentration of a) main polymer linkage b) oxygen with depth at different time and $x = 64\ \mu$m, in accordance with an embodiment of the present disclosure.

FIG. 11 depicts a graphical representation illustrating validation of concentration profile of carboxylic acid from a 2-D model with experimental results, in accordance with an embodiment of the present disclosure.

FIG. 12 depicts a graphical representation illustrating a surface roughness profile with time of the coating type, in accordance with an embodiment of the present disclosure.

FIG. 13 depicts a graphical representation illustrating a thickness profile with time of the coating type, in accordance with an embodiment of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Coatings or coating materials (e.g., paints, and the like) offer dual benefits of aesthetics and protection of the substrate on which it is applied. Coatings can protect the substrate from outside environmental agents like rain, sunlight, corrosion inducing agents, acid, dust, etc. A key part of paint formulations and development is to predict the service life of paints and maximize it. Everyday new coating formulations are developed, and they need to be tested rigorously under extreme and real weather conditions to predict the service life of the coatings before they can be released in the market. A model for predicting the service life or change in important coating characteristic/parameters over time can save time, efforts, and cost for releasing new formulated products in the market.

Most techniques to estimate the service life of coatings are experimental in nature. Experimental testing of coatings is expensive (destructive in nature) and time consuming (~5 years under natural conditions and few weeks under accelerated conditions). Very few models are available in literature (in research phase) to predict change in few physical properties (Martin et al, 1988 and Hinderliter et al, 2005) and chemical composition (Kiil, 2012) over time, but are limited in terms of the properties (either physical or chemical composition, not both) it can estimated and limited to the type and composition of coating. There are no models to understand the effects of change in concentration of the polymer due to weathering in two-dimensions (depth and length - also referred as 2D) and 3-dimensions (depth, length, and breadth - also referred as 2D) of the coating and correlate it to physical properties.

A physics-based model to predict the coating parameters during its weathering reduces the dependence on cumbersome and time-taking experimental methods used for determination. This leads to a drastic reduction of time from years to days to understand the effect of weathering on common parameters of paints like chemical composition, surface roughness, thickness, and gloss. Reduction of the need for experiments also leads to a reduction in costs as the experiments are destructive in nature. Embodiments of the present disclosure provide system and method that consider the effects of change in concentration of the polymer due to weathering and correlate the changes in chemical composition to physical changes like surface roughness and thickness reduction. This enables the system and method of the present disclosure to predict changes in physical characteristics of the coating due to changes in chemical composition in the presence of different environmental factors in full 3-D domain.

Referring now to the drawings, and more particularly to FIGS. 1 through 13, where similar reference characters denote corresponding features consistently throughout the figures, there are shown preferred embodiments and these embodiments are described in the context of the following exemplary system and/or method.

FIG. 1 depicts an exemplary system 100 for predicting properties of coating types and estimating service life thereof, in accordance with an embodiment of the present disclosure. In an embodiment, the system 100 may also be referred as 'material properties prediction system', 'service life estimation system' or 'material properties prediction and service life estimation system (MPPSLES) and interchangeably used herein. In an embodiment, the expression 'coating type' may also be referred as 'coating material' or 'coating' and interchangeably used herein. In an embodiment, the system 100 includes one or more hardware processors 104, communication interface device(s) or input/output (I/O) interface(s) 106 (also referred as interface(s)), and one or more data storage devices or memory 102 operatively coupled to the one or more hardware processors 104. The one or more processors 104 may be one or more software processing components and/or hardware processors. In an embodiment, the hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is/are configured to fetch and execute computer-readable instructions stored in the memory. In an embodiment, the system 100 can be implemented in a variety of computing systems, such as laptop computers, notebooks, hand-held devices (e.g., smartphones, tablet phones, mobile communication devices, and the like), workstations, mainframe computers, servers, a network cloud, and the like.

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) can include one or more ports for connecting a number of devices to one another or to another server.

The memory 102 may include any computer-readable medium known in the art including, for example, volatile memory, such as static random-access memory (SRAM) and dynamic-random access memory (DRAM), and/or non-volatile memory, such as read only memory (ROM), erasable programmable ROM, flash memories, hard disks, optical disks, and magnetic tapes. In an embodiment, a database 108 is comprised in the memory 102, wherein the database 108

comprises information pertaining to various coating types, substrates on which the coating types is/are going to be applied, weathering conditions that the coating type is to be subjected in one or more environments (e.g., summer, rainy, cloudy, snowy, adverse weathering conditions, and the like). The memory 102 further comprises one or more chemical-physical correlator(s), one or more partial differential equation (PDE) solvers, one or more optimizers, and the like. The memory 102 further comprises (or may further comprise) information pertaining to input(s)/output(s) of each step performed by the systems and methods of the present disclosure. In other words, input(s) fed at each step and output(s) generated at each step are comprised in the memory 102 and can be utilized in further processing and analysis.

FIG. 2, with reference to FIG. 1, depicts an exemplary high level flow diagram of the system for predicting properties of coating types and estimating service life thereof, in accordance with an embodiment of the present disclosure. FIG. 3, with reference to FIG. 1 through 2, depicts an exemplary high level flow diagram of the system 100 for predicting properties of coating types and estimating service life thereof, in accordance with an embodiment of the present disclosure.

FIG. 4, with reference to FIG. 1 through 3, depicts an exemplary flow chart illustrating a method for predicting properties of coating types and estimating service life thereof, using the system 100 of FIG. 1, and the exemplary flow diagrams of the FIGS. 2-3, in accordance with an embodiment of the present disclosure. In an embodiment, the system(s) 100 comprises one or more data storage devices or the memory 102 operatively coupled to the one or more hardware processors 104 and is configured to store instructions for execution of steps of the method by the one or more processors 104. The steps of the method of the present disclosure will now be explained with reference to components of the system 100 of FIG. 1, flow diagrams depicted in FIGS. 2-3, the flow diagram as depicted in FIG. 4, and graphical representations from FIG. 5A through 13.

In an embodiment, at step 202 of the present disclosure, the one or more hardware processors 104 obtain a coating type, and one or more weathering conditions that the coating type is to be subjected in an environment. The system 100 may be configured with one or more interfaces through which one or more inputs may be obtained. In an embodiment, information pertaining to coating type(s) (e.g., paint(s)) whose weathering phenomena is to be studied/analysed may be obtained as input by the system 100. There can be different classes of coating types (e.g., paints) from which it can be chosen as input. For instance, in the interface of the system 100 there may be an option for one or more users to select coating type wherein coating type such as wall paints, automotive paints, protective paints, special purpose paints, industrial paints, etc. may be selected in the system 100 for further analysis. Additional information may also be fed in the system 100 such as weathering condition in which the coating type will be subjected to and under which one or more degradation parameters have to be estimated. Weathering condition may include, but are not limited to, artificial or natural weathering conditions for studying weathering phenomena. For natural weathering, ambient environment conditions to which the coating type will be subjected is (or may) also be provided as input in terms of average temperature, average intensity of light, humidity and other relevant conditions. The coating type and the environmental conditions under which weathering happens are passed/processed for further information. In the present disclosure, specific case study, 'automotive coatings' has been selected as the model paint type. Such example of selection of the automative coating shall not be construed as limiting the scope of the present disclosure. Automotive coatings are subjected to Florida testing for ~10 years before they are commercially available, hence, models/systems (e.g., system 100) for predicting the rate at which the paints weathers in terms of thickness reduction, gloss loss and change in chemical composition can reduce the time required for testing significantly. The type of weathering conditions is chosen as artificial weathering conditions in which effects of only Ultraviolet (UV) light has been considered and all other factors are assumed to not take part in weathering process for the case study. Automotive coating and artificial weathering conditions are given as input for further processing.

Referring to steps of FIG. 4, in an embodiment, at step 204 of the present disclosure, the one or more hardware processors 104 identify a polymer for the one or more weathering conditions for the coating type. More specifically, the system 100 comprises the database 108 (e.g., also known as knowledge base) which takes the above input as specified in step 202. Using the input of step 202 along with literature information comprised in the database 108, the system 100/hardware processors 108 identify relevant information. More specifically, the hardware processors 104 search for a main polymer or binder which is primarily used in the type of coating (e.g., the automative paint) serving as the input. The database 108 provides quantitative values for the weathering parameters like light intensity, concentration of oxygen in atmosphere, average moisture content, initial thickness of the coating etc. and is used for further processing.

For example, in case of automotive coatings, the main polymer is identified as polyurethane since it is the most widely used type of base polymer and binder used in both base coat and clear coat which is the one most prone to weathering (refer FIG. 2 which depicts polyurethane being identified). The values of initial thickness of the automotive coatings are also identified from the database 108 and is taken to lie in the range of 100-200 $\mu$m. Values of the light intensity used in the artificial weathering conditions have also been identified from the database 108. The typical light intensity in artificial weathering conditions is taken to be 30 W/$m^2$ and the partial pressure of the oxygen is taken same as that of normal atmospheric conditions is 21278 Pa (21 vol% in air), in an example embodiment of the present disclosure. The concentration of the oxygen at the top surface of the coating exposed to environment is dependent on the polymer

properties i.e., solubility of oxygen in the polymer and is taken from the database 108 (e.g., say a polymer repository comprised in the database 108). The diffusivity of oxygen is also taken from the polymers repository based on the type of polymer, in absence of any specific value, wherein a generic value in the range of (1-0.4) $\times 10^{-13}$ $m^2/s$ has been used by the system and method of the present disclosure. The output of step 204 is the main binder/polymer and coating thickness with the quantitative values of environment conditions based on the selected weathering conditions for the coating type (e.g., automative paint).

Referring to steps of FIG. 4, in an embodiment, at step 206 of the present disclosure, the one or more hardware processors 104 identify one or more relevant reactions based on the identified polymer and the one or more weathering conditions. More specifically, at step 206, one or more compressed chemical reactions are identified based on the identified polymer (e.g., polyurethane). The system 100 (or the one or more hardware processors 104) search the polymer properties repository (comprised in the database 108) based on the polymer identified from the database 108. The system 100 identifies the initial concentrations (cross-link densities) of all the corresponding reactions, its kinetics, their reaction constants and other parameters like solubility and diffusion of oxygen in coating matrix, partial pressure of oxygen, spectral sensitivity of light absorbing component, etc. used in a reactions system. The polymer properties repository populates all values in form of table by first identifying a total number of components involved in the relevant reactions and identifying the reaction/source terms, diffusion term and convection term of each component with their corresponding boundary and initial conditions are taken from the polymers repository. Once all the terms are identified, values of the constants are again searched in the polymer repository for their approximate values and input as quantitative values in the table which is processed for further analysis. In the present disclosure, the system 100 takes polyurethane coating as the input and the environment conditions the coating type will be subjected to. All the reactions which are involved in the polyurethane degradation in the presence of UV radiation are taken from polymer properties repository. The complex reaction scheme of the polyurethane with UV light to end products are condensed in six reactions for polyurethane in the repositories and are presented in reaction 1-6. (Compressed chemical reactions).

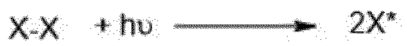

(R1)

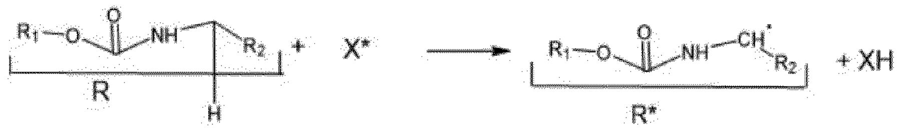

(R2)

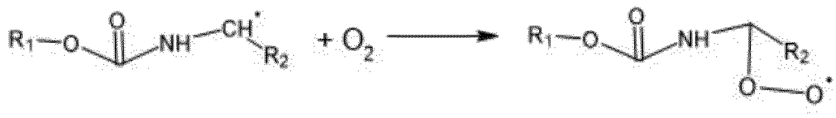

(R3)

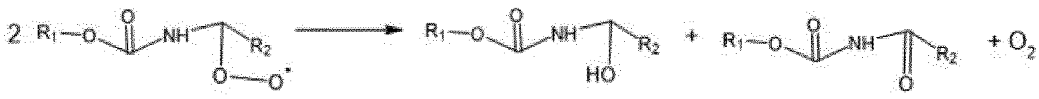

(R4)

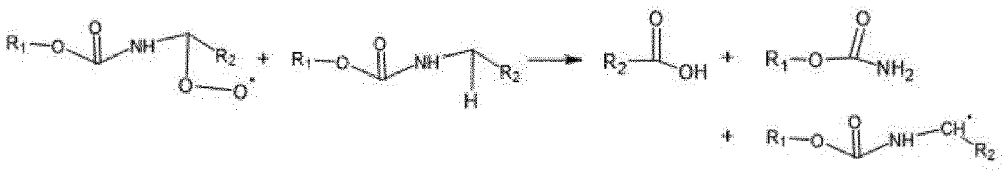

(R5)

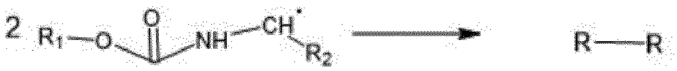

(R6)

Table 1 - Components involved in the reaction with UV

| Sl. No | Functional Group | Diffusion term | Source term | Convection term | Initial and boundary conditions |
|---|---|---|---|---|---|
| 1 | Main polymer - $R_1COONHCH_2R_2$ $[RH]$ | - | $-k_2[1][3]$; $-k_5[1][6]$ | - | Initial cross-link density |
| 2 | Impurity $[X-X]$ | - | -phi*eps*[2]*$I_0$*e$^{(-eps*l*[2])}$; $k_2[1][3]$ | - | 5% of cross-link density |
| 3 | Impurity radical- $[\dot{X}]$ | - | 2*phi*eps*[2]*$I_0$ *e$^{(-eps*l*[2])}$; $-k_2[1][3]$ | - | 0 |

| | | | | | |
|---|---|---|---|---|---|
| 4 | Main radical-$R_1COONHCH^*R_2$ $[\dot{R}]$ | - | $k_2[1][3]$; $-k_3[4][5]$; $-k_6[4][4]$ | - | 0 |
| 5 | Oxygen - $[O_2]$ | $D_{O2}del^2[5]$ | $-k_3[5][4]$; $k_4[6][6]$ | - | (x, t=0) - 0 (x=0, t) - $Seq \times P_{O2}$ (x=l, t) = del[5]/del x = 0 |
| 6 | Peroxy radical-$R_1COONHCOO^*R_2$ $[R\dot{O}O]$ | - | $k_3[5][4]$;$-k_5[6][1]$; $-k_4[6][6]$ | - | 0 |
| 7 | Alcohol-$R_1COONHCHOHR_2$ $[ROH]$ | - | $k_4[6][6]$ | - | 0 |
| 8 | Ketone-$R_1COONHCOR_2$ $[RO]$ | - | $k_4[6][6]$ | - | 0 |
| 9 | Carboxylic acid - $R_2COOH$ $[R_2COOH]$ | - | $k_5[6][1]$ | - | 0 |
| 10 | Urethane product – $[R_1COONH_2]$ | - | $k_5[6][1]$ | - | 0 |
| 11 | Crosslinking – $[R-R]$ | - | $0.5^*k_6[4][4]$ | - | 0 |

Polymer properties repository (refer circular depiction in FIG. 2): This repository is comprised in the database 108 (or the memory 102) and contains all the information about the different polymer systems, their reactions in presence of different environment agents, initial cross link concentration and their respective reaction kinetics and constants. The repository gives all this information for further processing in form of table with reactions as source terms.

For example, the system 100 takes the name of the polymer and the environment conditions as input and searches for the reaction mechanism corresponding to the polymers and environment conditions. Based on the input of polyurethane type of polymer in the presence of UV radiations a reaction scheme of 6 simplified reactions have been identified from the repository. The UV degradation of polyurethane is a complex process, consisting of many reactions in series and parallel. From the complex reaction mechanism, a simplified set of 6 simple reactions has been identified which can accurately represent the full degradation of polyurethane in presence of UV. Polyurethanes are not photoactive i.e., in

the presence of photons polyurethane do not generate radicals, but once radicals are generated, the polyurethane reacts with radicals to form degradation products.

The presence of impurities or residual solvents or pigments break down to form radicals in the presence of photons/light (represented by reaction R1). The radicals thus generated start the reaction scheme by firstly reacting with the polyurethane chain, leading to formation of urethane radical, represented in R2. The urethane radical thus formed reacts with oxygen to form peroxy radical (reaction R3). Peroxy radical is the one which is highly responsible for the degradation of the polymer matrix, as discussed in various literature. The peroxy radical thus formed leads to scission of the polyurethane linkage to carboxylic acid and urethane products. Also, peroxy radical may react with another peoxy radical to form aldehydes and ketones. The polyurethane radicals often combine with itself to form cross-linking, which is reported to increase brittleness in the coating. Aldehydes, ketones, carboxylic acids, cross-linker products, and urethane products are thus the degradation products of polyurethane in presence of UV. All the corresponding kinetics and parameters associated therein are further processed and analysed as mentioned below. The system 100 also identifies the solubility of oxygen in polymer matrix, diffusion of oxygen in polymer and the initial cross-linking density of the polyurethane. All the values of the constants are presented in Table 2 in the SI units and is given as output to form a table with quantitative values to solve the reaction scheme.

Table 2 - Values of parameters in SI unit from the repository

| Parameter with units | Description | Values |
|---|---|---|
| $k_1$ $(m^2/W)s^{-1}$ | rate constants for initiation of radical from impurities | 1 (assumed, adjustable) |
| $k_2(m^3/mol)$ $s^{-1}$ | rate constant for propagation to urethane linkage | 0.1 |
| $\varphi$ (dimensionless) | Quantum yield of urethane resin | $4 \times 10^{-6}$ |
| $E_0$ $(W/m^2)$ | Radiation intensity at coating surface | 30 |
| $\alpha_h$ $(m^2/mol)$ | Spectral sensitivity of radical formation | 1 |
| $k_3$ $(m^3/mol)$ $s^{-1}$ | Rate constant for formation of peroxy radical | 10 |
| $k_4$ $(m^3/mol)$ $s^{-1}$ | Rate constant for termination reaction of peroxy | 0.1 |
| $k_5$ $(m^3/mol)s^{-1}$ | Reaction of peroxy with urethane linkage/ scission of polyurethane | 0.1 |
| $k_6$ $(m^3/mol)$ s | Rate constant for cross linking | 1 |
| $[RH_0]$ mol /($m^3$ coating) | Initial concentration of polymer matrix | 4082 |
| $D_{O_2}$ $(m^2/s)$ | Diffusivity of oxygen in film | $7.5 \times 10^{-13}$ |
| $S_{eq}$ (mol/$m^3$ coating Pa) | Solubility of oxygen in film | $1.6 \times 10^{-4}$ |
| $P_{O_2}$ (Pa) | Partial pressure of oxygen in air (conditions) | 21278 |

Referring to steps of FIG. 4, in an embodiment, at step 208 of the present disclosure, the one or more hardware processors 104 estimate a mass balance equation for each chemical component comprised in the one or more relevant reactions (e.g., the one or more compressed chemical reactions). The above step of 208 is better understood by way of following description: Material balance of all the different compounds identified in the reaction mechanism, reactants as well as products are completed by the system 100 (or the one or more hardware processors 108) at step 208. A material balance based on conservation of mass for any compound can be given as - Accumulation/Unsteady state term = Diffusion +Source - Convection.

The material balance in this case is generally done with respect to concentration. The unsteady source term in this case will be a partial differential operator since the change in concentration is with both time and space. Material balance is done for all the components and a system of Partial Difference equations (PDEs) is generated with boundary and initial conditions known for all individual equations. The formulated system of PDEs is output from the system 100 (e.g., refer material balance block in FIG. 2 and PDE solvers blocks in FIG. 3. In the present disclosure, for the case study of automative paints and the identified polymer (e.g., polyurethane), a total of 11 components involved in the reaction mechanism (e.g., compressed chemical reactions) for polyurethane degradation have been identified from polymer property repository wherein these include, but are not limited to, reacting species (main polymer linkage, impurity, oxygen), intermediates (impurity radical, main radical, peroxy radical) and products (alcohol, ketone, carboxylic acid, urethane product, cross-linking product). All the respective transient terms, diffusion terms, source term and convection terms have also been identified based on the information about reaction kinetics for polyurethane degradation in the presence of only ultraviolet (UV) light. For individual terms, their respective constants like diffusivity of oxygen, rate constants, molar

absorptivity and intensity of the light were input based on the environment and the type of coating obtained from the database 108.

For polyurethane degradation by UV, the complex reaction scheme was condensed into a set of 6 major reactions, starting from initiation of degradation to final volatile and cross-linked products. A total of 11 compounds were involved in the 6 reactions, in which 3 were reactants and the other 8 were identified as products or intermediates. The diffusion terms, source (reaction) term and convection for each component are inputted from the Table 1 with corresponding equation and values of constants. In the present case, diffusion is only because of the oxygen component, and water has not been included in the reaction system. Rest, all the components are solids and hence the diffusion term is zero. Moreover, there is no convection inside the coating matrix and hence, the terms corresponding to convection are zero for all components in the reaction. Material balance of 11 compounds led to formation of a system of 11 partial differential equations. Each equation has corresponding/associated initial condition(s) and boundary conditions. The initial condition is at time t=0 i.e., when no reaction has started and boundary condition will be at two locations y=0, which is the top surface of the coating and y=l, where 'l' is the total depth of the coating. For all the intermediate and products, the initial conditions are equal to zero at time t=0. Also, the boundary conditions for all the solid components (functional groups) where there is no diffusion, boundary conditions are not needed. The initial condition of the two reactants- main polyurethane (also referred as main polymer and interchangeably used herein) linkage and impurity are obtained from the database 108. For main polyurethane linkage, initial concentration is equal to the cross-link density of the polyurethane since polyurethane linkage is caused during the crosslinking. The impurity in the polymer matrix which initiates the degradation reaction by generation of radicals by action of UV light is assumed to have an initial concentration of p% (e.g., p=1) of the main polyurethane linkage. For oxygen, the initial concentration for oxygen is zero in the bulk of coating. For oxygen, at the top surface of the coating, there is constant value boundary condition and at the base of the coating (y=l) no penetration boundary condition was considered. The boundary condition for oxygen can be defined as-

$$\text{at } y = l, \frac{\partial [O_2]}{\partial y} = 0$$

$$y{=}0 \; [O_2] = S \times P_{O_2}^{air}$$

where $S$ is the solubility of oxygen in the coating and $P_{O_2}^{air}$ is partial pressure of oxygen in air. The PDE for oxygen after material balance with its boundary conditions and initial condition can be shown as -

$$\frac{\partial [O_2]}{\partial t} = \frac{\partial}{\partial y}\left(D_{O_2}\frac{\partial [O_2]}{\partial y}\right) - k_3[\dot{R}][O_2] + k_4[R\dot{O}O]^2$$

$$[O_2](t = 0, y) = 0$$

$$[O_2](t, y = 0) = S \times P_{O_2}^{air}$$

$$\frac{\partial [O_2]}{\partial y}(t, y = l) = 0$$

Also, for different other 10 components, the PDEs with initial conditions can be represented as -

$$\frac{\partial [RH]}{\partial t} = -k_2[\dot{X}][RH] - k_5[R\dot{O}O][RH]; \; [RH](t = 0, y) = [RH]_0$$

$$\frac{\partial [X - X]}{\partial t} = -k_1\varphi[X - X]I_0 e^{(-\alpha y[XH])} + k_2[\dot{X}][RH]; \; [XH](t = 0, y) = 10\%([RH]_0)$$

$$\frac{\partial[\dot{X}]}{\partial t} = 2k_1\varphi[X - X]I_0e^{(-\alpha y[XH])} - k_2[\dot{X}][RH];\ [\dot{X}](t = 0, y) = 0$$

$$\frac{\partial[\dot{R}]}{\partial t} = k_2[\dot{X}][RH] - k_3[\dot{R}][O_2] - k_6[\dot{R}]^2;\ [\dot{R}](t = 0, y) = 0$$

$$\frac{\partial[R\dot{O}O]}{\partial t} = k_3[\dot{R}][O_2] - k_4[R\dot{O}O]^2 - k_5[R\dot{O}O][RH];\ [R\dot{O}O](t = 0, y) = 0$$

$$\frac{\partial[ROH]}{\partial t} = k_4[R\dot{O}O]^2;\ [ROH](t = 0, y) = 0$$

$$\frac{\partial[RO]}{\partial t} = k_4[R\dot{O}O]^2;\ [R\dot{O}O](t = 0, y) = 0$$

$$\frac{\partial[R_2COOH]}{\partial t} = k_5[R\dot{O}O][RH];\ [R_2COOH](t = 0, y) = 0$$

$$\frac{\partial[R_1COONH_2]}{\partial t} = k_5[R\dot{O}O][RH];\ [R_1CONH_2](t = 0, y) = 0$$

$$\frac{\partial[R - R]}{\partial t} = 0.5k_6[\dot{R}]^2;\ [R - R](t = 0, y) = 0$$

The values of all the constants from diffusivity of oxygen to reaction constants and molar absorptivity are taken from the repository/database 108 and are inputted as is at step 208. The intensity of the light depends on the environment it is subjected to, and the average intensity of light experienced by that geographical area. The formulated set of PDEs are further processed for solving to generate useful results.

In an embodiment, at step 210 of the present disclosure, the one or more hardware processors 104 solve the mass balance equation for each chemical component to (i) predict one or more properties of the coating type, and (ii) obtain an estimated service life of the coating type. The service life of the coating type (e.g., service life of paint) refers to a time limit or time range beyond which the coating type/paint is not recommended for use, or the coating type/paint is not usable for applications. The above step of 210 is better understood by way of following description:

The formulated set of PDEs are processed for solving and obtaining the results, concentration profiles, gloss loss, thickness loss, mass loss as output(s). For the case study of polyurethane coating, all the corresponding equations and terms serve as input for all 11 compounds, the set of 11 PDE equations with their corresponding initial and boundary conditions are processed. More specifically, the step of solving the mass balance equation for each chemical component comprises processing the mass balance equation for each chemical component by a first Partial Difference Equations (PDE) solver to obtain (i) a first concentration profile, and (ii) a second concentration profile comprising an associated depth for each chemical component. Further, by using a first chemical-physical correlator, a first set of chemical-physical correlations of constituents comprised in the coating type is estimated based on (i) the second concentration profile comprising the associated depth and (ii) time at which the coating type is applied and (iii) the one or more weathering conditions that the coating type is to be subjected in the environment. Further, an optimizer (as known in the art optimizer and comprised in the memory 102 and when invoked for execution), uses (i) the second concentration profile comprising the associated depth and (ii) the time at which the coating type is applied and (iii) the one or more weathering conditions that the coating type is to be subjected in the environment and estimates one or more optimized reactions-based parameters. The one or more optimized reactions-based parameters are further processed via a second PDE solver and a third PDE solver to obtain a third concentration profile, and a fourth concentration profile, respectively. The third concentration profile further includes depth and length (2D). Similarly, the fourth concentration profile further includes depth, length, and breadth (3D). Furthermore, by using a second chemical-physical correlator, a second set of chemical-physical correlations

of constituents comprised in the coating type using at least one of the third concentration profile, and the fourth concentration profile.

The one or more hardware processors 104 further determine a change in surface roughness of the coating type with reference to time based on the second set of chemical-physical correlations of constituents comprised in the coating type.

The one or more hardware processors 104 further determine a change in thickness of the coating type with reference to time based on the first set of chemical-physical correlations and the second set of chemical-physical correlations of constituents comprised in the coating type.

The one or more hardware processors 104 further determine a change in (i) gloss, (ii) a Griffith's stress, and (iii) a wetting angle of the coating type based on the change in surface roughness of the coating type with reference to time.

The one or more hardware processors 104 further compute an estimated service life based on at least one of (i) the change in surface roughness of the coating type, (ii) the change in thickness of the coating type, (iii) the gloss, (iv) the Griffith's stress, and (v) the wetting angle of the coating type. The above various outputs are depicted in FIG. 2 and 3, respectively. Further, the above outputs computed as better understood by way of following description:

For instance, the step of processing the mass balance equation for each chemical component by a first Partial Difference Equations (PDE) solver to obtain (i) the first concentration profile, and (ii) a second concentration profile comprising an associated depth for each chemical component is described below. The first Partial Difference Equations (PDE) solver may also be referred as 1-D PDE model solver and interchangeably used herein. The 1-D PDEs Model solver solves the system of PDE equations with time in only one dimension of length and hence the term 1-D PDEs Model solver. This 1-D PDEs Model solver uses finite difference to solve the system of PDEs with all the specified boundary and initial conditions from obtained from the material balance block of FIG. 2. Based on the time for which the concentration profile or the effects of weathering are to be estimated the system of equations are solved for the total time. The time step is decided based on the resolution required in the coating matrix and the stability of the solution. The time step and the total time for which the simulation has to be carried out determines the simulation time. The time step is often of the order of millisecond and total time is in days and months. The concentration profiles generated along the depth of the coating are processed by the optimizer (refer FIG. 3 for optimizer) for matching with the experimental results and adjusting the kinetic parameters. The concentration profiles from the final set of optimized parameters are further processed for approximate results like thickness reduction with time and change in concentration profile with time.

For example, a set of 11 PDEs are to be solved with time, in one dimension which is the depth of the coating. The dimension along which the PDE equation is solved is the most important dimension since the oxygen concentration and light intensity varies with the depth of coating due to the diffusion process and Beer Lambert's law, respectively. Out of the 11 equations, only one equation, PDE of oxygen is to be solved with finite difference, because of the presence of diffusivity term making the differential equation in two variables. The discretization is carried out for the oxygen PDE in both space and time. The step size for the length and time are taken such that the solution remains stable. The step size of the length is determined by the number of discrete points, where concentration is to be estimated. The discrete number of points taken is 40 for 140 $\mu$m length of coating. For stable solutions, the time step is thus calculated as ~1 millisecond. Rest all the equations can be assumed as a set of ordinary differential equations (ODEs) with different equations along the length for the same compound at discrete 40 length points.

The oxygen equation is solved by the implicit finite difference method (as known in the art method) and rest all the equations are solved by the Runge-Kutta method (also referred as RK-4 - as known in the art method) for solving Ordinary Differential Equations (ODEs) at all discrete lengths, and wherever applicable the value of *x* at the discrete point was used in the equation. The final time for the simulation was kept at 80 days. This was done to compare the concentration profiles with the experimental data available in conventional research work (e.g., refer Larch et al (2011)), for change in functional groups absorbance over time for polyurethane coating in accelerated weathering conditions i.e., only UV at high intensity. The concentration profiles generated for each component over the length with varying time were stored for comparison with experimental results and estimating approximate output parameters. The concentration profile of all the components is shown in various FIGS. The concentration profile(s) is/are further processed for generating approximate results based on 1-D profile and for optimizing system parameters to predict weathering of paints/coating type closer to the experimentally observed weathering. The optimized parameters are also sent back the system 100 first PDE solver to obtain better concentration profiles which are subsequently used to obtain approximate weathering results like change in concentration with time and thickness reduction with time.

The above step of estimating, via the optimizer, by using (i) the second concentration profile comprising the associated depth and (ii) the time at which the coating type is applied and the one or more weathering conditions that the coating type is to be subjected in the environment, the one or more optimized reactions-based parameters is better understood by way of following description:

The kinetic parameters and other system parameters are obtained from the database 108. The parameters are collected for a system closest to the one of interests. Hence the parameters may not be available for a system identical to the one that was solved in the first PDE solver, which may lead to a loss in accuracy in predictions. Therefore, the parameters must be

adjusted for the system 100 before these are passed/processed for more accurate higher dimensional solvers. The results from the first PDE solver needs to be compared against results for a similar system from other sources (repository/database 108 or performing experiments). It is to be noted that it suffices even if the data from other sources are limited, or the experiments are performed for lesser duration of time. The system 100 attempts to minimize the error between the data from the simulations carried out by the method of the present disclosure simulations and the data from the other source by adjusting the parameters.

For example, the experimental data available in Larche et al (2011) is in terms of absorbance value for functional groups in question and hence the concentration profile of each functional group needs to be modified for comparison with the experimental results.

The concentration values at each point of length starting from surface (y=0) is integrated up to 20 points i.e., 70 μm since the FTIR waves can typically penetrate up to a length of 70 μm only.

$$A = K \int_{y=0}^{y=70} c(y)$$

where *A* is an absorbance of a particular functional group, *K* is the constant, and *c*(*y*) is concentration as function of depth which is integrated between 0 μm and 70 μm which represents top surface of the coating and depth of coating till Fourier-Transform Infrared Spectroscopy (FTIR) waves penetrate, respectively.

The absorbance of any functional group is proportional to the integration of the concentration up to the penetration depth of the wavelength which is approximately 70 μm. The absorbance and the integrated concentration were normalized with their initial values and were compared with experimental data to estimate mean-squared error (MSE). The products were initialized with main polyurethane initial value. The MSE value from the first set of parameters were at - 0.245. The parameters were adjusted in accordance with the experimental data and a new set of parameters were then given to 1-D PDE model solver to give accurate results. The step was repeated till MSE fell below a pre-defined value (0.0001). The simulation results were compared for both the reactants (main polyurethane linkage) and the products (carboxylic acid). The set of parameters which gives the lowest MSE for both the cases. The comparison with experimental results for main polymer linkage and carboxylic acid with corresponding MSE values for three different parameters set are shown in FIGS. 5A through 5C. More specifically, FIGS. 5A through 5C, with reference to FIGS. 1 through 4, depict a graphical representation illustrating a comparison of mean-squared error (MSE) estimated (i) during simulation by the system 100 and (ii) by conventional research work (Larche et al.), in accordance with an embodiment of the present disclosure. In FIGS. 5A through 5C, the MSE estimated are 0.07955, 0.05818, and 0.00543 respectively.

The optimized set of parameters were sent to 2-D PDE model solver and/or 3-D PDE model solver to estimate the weathering parameters across length and both length and breadth, respectively in addition to the depth of the coating, based on the requirements (e.g., requirements specified by one or more users).

The optimized set of parameters were also used to obtain concentration profiles from the 1-D PDE model solver for some approximate results. The concentration profiles with time at different lengths for main polyurethane linkage, oxygen, carbonyl, and carboxylic acid are depicted in FIGS. 6A through 6D. More specifically, FIGS. 6A through 6D, with reference to FIGS. 1 through 5C, depict a graphical representation illustrating a concentration profile of main polyurethane, in accordance with an embodiment of the present disclosure. In other words, FIGS. 6A through 6D depict representative concentration profile for reactants and products with time at different depths. As can be seen in FIGS. 6A through 6D, concentration of main polyurethane linkage at 0 μm (top surface of the coating) decreases the fastest and slowest at the substrate surface (y=l). This is because the light intensity and oxygen concentration are maximum at the top surface and minimum at the substrate surface. Similarly, most of the volatile products are shown to generate first at the top surface of coatings (y=0).

The concentration profile can be used to get meaningful insights of the change in concentrations of various functional groups with the depth of the coating and for knowing the time the coating takes to degrade based on the change in main polyurethane linkage concentration.

The step of estimating, by using the first chemical-physical correlator, the first set of chemical-physical correlations of constituents comprised in the coating type based on (i) the second concentration profile comprising the associated depth and (ii) time at which the coating type is applied and the one or more weathering conditions that the coating type is to be subjected in the environment, is better understood by way of following description:

The relationship between concentration and material ablation can be established, to estimate the change in macroscopical physical properties due to weathering with time. A cutoff concentration is decided for main polymer linkage, below which if the concentration drops, the material is assumed to ablated at that point. This assumption is based on the fact that as the concentration proceeds, the main polymer is converted into volatile products and hence the material ablation happens.

For the case study as mentioned by the system and method of the present disclosure, it was assumed that once the concentration of main polyurethane linkage at any length drops to z% (e.g., 10%) of the initial concentration, that

surface is ablated. This assumption is in accordance with the fact that once the main polyurethane reacts, the final products are volatile in terms of carbonyls and aldehydes. These volatiles tend to escape into the environment, causing ablation of the coating surface. The system takes information about thickness variation with time wherein the thickness profile with time can then be used to predict service life of the paints, knowing the relationship that if the thickness goes below a certain value(s) the paint is said to be unserviceable (or not usable). An approximate thickness profile with time is depicted in FIG. 7 for predicting thickness reduction during weathering. More specifically, FIG. 7, with reference to FIGS. 1 through 6D, depicts a graphical representation illustrating thickness reduction with time from 1-D space for the coating type, in accordance with an embodiment of the present disclosure.

The step of processing the one or more optimized reactions-based parameters via the second PDE solver and the third PDE solver to obtain a third concentration profile, and a fourth concentration profile, respectively is better understood by way of following description:

The system of PDEs, obtained from the material balance block of FIG. 2, is solved using a hybrid approach (RK-4 (fourth order explicit Runge-Kutta method that requires the same number of steps as the order of accuracy) + Lattice-Boltzmann (LB)) with the optimized set of parameters obtained from the optimizer by the second PDE solver (also referred as 2-D PDE solver). If concentration profile is solved only along the coating depth direction, it will not be able to capture concentration evolution along the surfaces at different depth, which can give intuition about the surface roughness. Surface roughness is a very important parameter to monitor degradation of paints and coatings as it can used to describe loss of macroscopic properties like gloss and can be used to find regions of stress concentration which eventually leads to crack creation and propagation.

Similarly, the system of PDEs, obtained from the material balance block of FIG. 2, is solved using a hybrid approach (RK-4 + Lattice-Boltzmann (LB) method) with the optimized set of parameters obtained from the optimizer by the third PDE solver (also referred as 3-D PDE solver). More specifically, solution of the system of PDEs in 3D domain adds on to the advantages of 2D domain by incorporating variation in concentration across all three dimensions. This naturally, results in the highest accuracy among the three solvers presented here. However, solving in 3D domain significantly adds to the computational costs. Therefore, benefits and risks of each model needs to be weighed against one or more objectives of interest to arrive upon the correct model to be used. The system and method therefore provide options to choose to solve the PDEs in 2D or 3D domain depending upon his/her accuracy requirements and available computational resources. In this case study, the system and method have opted to solve the system of PDEs in 2D domain. For experiments, the system and method assumed a cuboidal coating matrix where oxygen and U.V radiation are coming from bottom (e.g., refer FIG. 8). More specifically, FIG. 8, with reference to FIGS. 1 through 7, depicts a schematic of 2D simulations performed by the system 100 of FIG. 1, in accordance with an embodiment of the present disclosure. The evolution of polymer concentration across the whole 3D coating matrix domain can be obtained by solving full reaction network of polymer degradation along with diffusion of oxygen.

For a 1D model, described in the first PDE solver of FIG. 3, the variation in concentration only along the z-axis was considered. Here for 2D model, the system 100 considered variations in the x-axis as well (assuming no variation in the y-direction). In this setup, as oxygen is the only diffusing component, this can be used to present disclosure's advantage by solving ODEs for solids locally at each grid point and PDEs by LB method. For solving 2-D model by finite difference approach, the equation for oxygen must be discretized in multiple directions. Hence, the system 100 has used the LB method which reduces the effort of discretization as compared to finite difference technique.

For the system to be numerically stable, lattice timestep ($\tau$) of simulation should be taken as same as RK-4 method stability timestep since both are used simultaneously. Lattice diffusivity $D_L$ is set as from a stable choice of $\tau$ since $\tau$ *is* related to $D_L$. From $D_L$, temporal resolution in a case can be derived where spatial resolution and diffusivity of oxygen in the coating matrix are fixed. The spatial resolution is assumed to be $1\mu m$ and with stable lattice diffusivity 4.9 and real diffusivity of oxygen $7.5\times10^{-13}$ $m^2/s$ timestep comes out to be 0.001 second.

However, coating degradation is an inherently stochastic process. Even if exposed to the same conditions, coating over a surface does not degrade uniformly. After exposure to weathering conditions for a long period of time, the surface contains patches of coating with different levels of degradation. To model the difference in weathering extent over the same depth is quite difficult using deterministic modelling techniques since the oxygen concentration and light intensity for a surface at a particular depth are same. Hence, the system and method of the present disclosure approached this problem where the molar absorptivity ($\phi$) value of the binder polymer was varied across the entire domain leading to different weathering levels at one depth. The difference due to oxygen diffusion and light intensity penetration remains intact.

$$\phi(i,j) = \phi_m + R(i,j) * (\phi_{max} - \phi_{min})$$

Therefore, the system 100 randomizes the rate of the initiation reaction which leads to micro-randomness in the concentration profile. The concentration profiles still have a fixed pattern on a broader scale due to the PDEs. Physically,

the randomness in molar absorptivity represents unequal effect of weathering conditions on the same surface, which leads to a rough surface. However, the deterministic nature of the PDEs still have a significant effect on the concentration profile, as there should be more degradation near the edge exposed to the weathering elements and gradually decrease with coating depth. The concentration profiles can be processed to generate output visualizing concentration profiles at different time steps. Further, the concentration profiles generated can be processed to correlate concentration with macroscopic physical properties. Therefore, the output of the step of processing the one or more optimized reactions-based parameters via the second PDE solver and the third PDE solver with the combined LB and RK-4 method gives concentration profiles of different components with time along the x and y-direction of the coating. The concentration profiles can be obtained for different time instances. For the case taken, concentration profiles for main polymer linkage are shown at two different time steps along x and y-direction. y-direction being the depth of the coating. The depth and length of the coating for 2-D model is assumed to be 64 $\mu$m and 128 $\mu$m, respectively. FIGS. 9A and 9B, with reference to FIGS. 1 through 8, depict length of coating in $\mu$m, in accordance with an embodiment of the present disclosure. More specifically, FIGS. 9A and 9B depict normalized RH concentration contour after 8.33 mins and 16.66 mins with random absorptivity ($\phi$). Also, for representation normalized concentration of main polymer linkage and oxygen at x = 64 $\mu$m with depth is shown for 3 different time instances in FIGS. 10A and 10B. More specifically, FIGS. 10A-10B, with reference to FIGS. 1 through 9B, depict a graphical representation illustrating a normalized concentration of a) main polymer linkage b) oxygen with depth at different time and x = 64 $\mu$m, in accordance with an embodiment of the present disclosure. The concentration profile of product carboxylic acid was validated with experimental results and the same is presented in FIG. 11. More specifically, FIG. 11, with reference to FIGS. 1 through 10B, depicts a graphical representation illustrating validation of concentration profile of carboxylic acid from a 2-D model with experimental results, in accordance with an embodiment of the present disclosure.

The step of estimating, by using the second chemical-physical correlator, the second set of chemical-physical correlations of constituents comprised in the coating type using at least one of the third concentration profile, and the fourth concentration profile and determining the change in surface roughness of the coating type with reference to time based on the second set of chemical-physical correlations of constituents comprised in the coating type are better understood by way of following description:

After solving the set of PDEs, the concentration profiles of different components in the system 100 are obtained. The system 100 analyzes this data to predict the changes in the macroscopic properties (like thickness loss, mass loss, gloss loss, increase in hydrophilicity or loss of hydrophobicity, decrease in fracture toughness, etc.) of the coating matrix. The root cause of these changes in macroscopic properties is the removal of the binder polymer from the coating. The method of FIG. 4 is performed by the system 100 to connect the chemical degradation of binder polymer to the changes in macroscopic properties on the basis of cutoff concentration. Once the chemical concentration profile of the binder polymer (RH, in this case study) is obtained, its concentration was related to the macroscopic properties using the following condition. When the concentration of RH at a particular location falls below 15% of its initial concentration (i.e., more than 85% conversion), the polymer was considered for removal from that point. Here, it is assumed by the system and method of the present disclosure that the degradation products are volatiles and hence, by removal of the polymer from a location, complete removal of material from that location is implied. It is expected that the coating front most exposed to the weathering elements suffers the most from material removal, or ablation, and this ablated front moves forward along the coating depth, as time passes (leading to thickness loss, mass loss and increase in hydrophilicity or loss of hydrophobicity). Due to introduction of micro-randomness in the system in terms of absorptivity, the ablated front is not a smooth surface. The roughness stems from the uneven degradation of the polymer across the coating matrix. Increase in surface roughness results in loss of gloss and increase in hydrophilicity or loss of hydrophobicity.

The final physical coating surface at different degradation times, is passed/processed for predicting the surface roughness of the coating. As mentioned earlier, surface roughness arises from the uneven degradation of the surface and can be computed by the thickness at every point of length (x). The surface roughness profile with time can then be used to predict service life of the paints, knowing the relationship that if the surface roughness goes above a certain value the paint is said to be unserviceable.

Data such as thickness at every point in x-direction, based on, till what depth the material has ablated is obtained. Normalized thickness ($t_i$) at every point i in x-direction is given by:

$$t_i = y_i / L_o$$

where $y_i$ is the depth of coating at every point '*i*' in x-direction and hence is function of *x* and $L_o$ is an initial length of the coating. The number of points i where depth of coating is calculated is dependent on the spatial resolution. The mean normalized thickness can thus be determined from the equation:

$$t = \sum_{i} t_i$$

And the surface roughness of the exposed surface is thus defined by

$$Roughness(R) = (1/N) * \sqrt{\sum (t_i - t)^2}$$

Where $N$ is the total number of grid points ($i$).

Macroscopic properties are computed after the rough surface is generated. A change in thickness of the coating type is determined with reference to time based on the first set of chemical-physical correlations and the second set of chemical-physical correlations of constituents comprised in the coating type. The thickness of the coating is measured as the mean of the heights at each grid point across the domain, with its standard deviation being computed as a measure of surface roughness. This is the first attempt to predict surface roughness from first-principal models. The surface roughness thus calculated with time is sent to the user as output and the same is depicted in FIG. 12. FIG. 12, with reference to FIGS. 1 through 11, depicts a graphical representation illustrating a surface roughness profile with time of the coating type, in accordance with an embodiment of the present disclosure.

The data of thickness ($y$) at different $x$ can be processed further to predict the thickness reduction in addition to the surface roughness. The thickness profile with time can then be used to predict service life of the paints, knowing the relationship that if the thickness goes below a certain value the paint is said to be unserviceable. The scaled thickness can be calculated with value of $y_i(x)$ at different $x$ according to equation:

$$t = \frac{1}{N}\left(\sum_{i} \frac{y_i}{L_0}\right)$$

The scaled thickness profile with time is presented in FIG. 13. More specifically, FIG. 13, with reference to FIGS. 1 through 12, depicts a graphical representation illustrating a thickness profile with time of the coating type, in accordance with an embodiment of the present disclosure. Estimates of other macroscopic properties can be obtained by using the following relations. The gloss can be predicted using surface roughness according to the following equation:

$$R_s = R_0 e^{-\frac{(4\pi\sigma)^2}{\lambda^2}}$$

where $R_0$ (dimensionless) is reflectance of a perfectly smooth surface of same material, $\lambda(m)$ is wavelength of light and $\sigma$ ($m$) is surface roughness. The change in gloss with time thus can be found out if the change in surface roughness with time is known. Also, the change in griffith's stress of the material and wetting angle can be predicted by the following equations:

$$\sigma_G = \sqrt{\frac{2E\gamma_s}{\pi a}}$$

where $\sigma_G$ is Griffith's stress, E is Young's Modulus, $\gamma_s$ is surface energy per unit area and '$a$' is maximum flaw depth.

$$\cos\theta = r\left(\frac{\gamma_{SV} - \gamma_{SL}}{\gamma_{LV}}\right)$$

where $\gamma$ is surface tension between two interfaces, $\theta$ is wetting angle and '$r$' is ratio of actual area and projected area on the surface. The gloss reduction profile with time can then be used to predict service life of the coating type (e.g., paints), knowing the relationship that if the gloss goes below a certain critical value of gloss, the coating type (e.g., paint) is said to be unserviceable. The service life of paints can be calculated by the following formula:

Service life = (Total life of the coating based on the minimum/critical value of the parameter - Actual number of years remaining based on the current value of the parameter).

Most techniques to estimate the service life of coatings are experimental in nature. Experimental testing of coatings is expensive (destructive in nature) and time consuming. Present disclosure provides system and method that predict change in chemical composition of the coating using reaction mechanism of the coating components in presence of weathering causing environmental agents. The method and system of the present disclosure considers the effects of change in concentration of the polymer due to weathering and correlate the changes in chemical composition to physical changes like surface roughness and thickness reduction. A complete model will thus be able to predict changes in physical characteristics of the paint due to changes in chemical composition in the presence of different environmental factors in full 3-D domain. The degradation mechanism and the rate equations, along with the kinetic parameters, are also estimated/known by performing various steps carried out by the method described herein. It is to be noted that development of such method/system is an interdisciplinary effort, needing expertise in chemical engineering, chemistry, programming, modelling, and mechanical engineering for prediction of surface roughness from reactions and chemical perspective. Generally, in prior arts, physical and chemical properties of paints/coatings have been modelled as different systems and no accurate correlation is available. Non-uniformity in coating degradation is captured by the system of the present disclosure by randomizing the molar absorptivity of photoactive components in the coating within a small range wherein the system of the present disclosure captures the inherent randomness/stochasticity in the weathering phenomenon. Among the many system parameters in weathering, finding the right parameter to randomize is a challenging task which is achieved by the system and method of the present disclosure. Coating degradation involves multiple reactions in series and parallel that requires understanding different degradation reaction mechanisms which is resolved by the system and method of the present disclosure.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments

consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

**1.** A processor implemented method for predicting properties of a coating type and estimating service life, the processor implemented method comprising:

obtaining, via one or more hardware processors, a coating type, and one or more weathering conditions that the coating type is to be subjected in an environment (202);
identifying, via the one or more hardware processors, a polymer for the one or more weathering conditions for the coating type (204);
identifying, via the one or more hardware processors, one or more relevant reactions based on the identified polymer and the one or more weathering conditions (206), wherein identifying the one or more relevant reactions comprises at least one of: identifying initial concentrations of the one or more reactions, kinetic parameters of the one or more reactions, one or more system parameters, reaction constants, solubility and diffusion of oxygen in coating type, spectral sensitivity of light absorbing component;
estimating, via the one or more hardware processors, a mass balance equation for each chemical component comprised in the one or more relevant reactions (208); and solving, via the one or more hardware processors, the mass balance equation for each chemical component to (i) predict one or more properties of the coating type, and (ii) obtain an estimated service life of the coating type (210), wherein the step of solving the mass balance equation for each chemical component comprises:

generating a concentration profile with a depth of the coating type;
receiving the generated concentration profile by Partial Difference Equations (PDE) solver for matching the kinetic parameters and the one or more system parameters;
optimizing the one or more system parameters for the generated concentration profile;
sending the optimized one or more system parameters to the first Partial Difference Equations (PDE) solver to obtain thickness reduction with time, change in the concentration profile with time, gloss loss, mass loss, wherein the one or more system parameters are processed to minimize errors in modeling by adjusting the one or more system parameters;
processing the mass balance equation for each chemical component by a first Partial Difference Equations (PDE) solver to obtain (i) a first concentration profile, and (ii) a second concentration profile comprising an associated depth for each chemical component;
estimating, by using a first chemical-physical correlator, a first set of chemical-physical correlations of constituents comprised in the coating type based on (i) the second concentration profile comprising the associated depth and (ii) time at which the coating type is applied and (iii) the one or more weathering conditions that the coating type is to be subjected in the environment;
estimating, via an optimizer, by using (i) the second concentration profile comprising the associated depth and (ii) the time at which the coating type is applied and (iii) the one or more weathering conditions that the coating type is to be subjected in the environment, one or more optimized reactions-based parameters;
processing the one or more optimized reactions-based parameters via a second PDE solver and a third PDE solver to obtain a third concentration profile, and a fourth concentration profile, respectively; and
estimating, by using a second chemical-physical correlator, a second set of chemical-physical correlations of constituents comprised in the coating type using at least one of the third concentration profile, and the fourth concentration profile, wherein

the service life of the coating type is estimated by using the one or more concentration profiles, by predicting changes in physical characteristics of the coating type due to changes in the chemical components in presence of the one or more weather conditions, wherein the changes comprises at least one of non-uniformity in coating degradation,

surface roughness, thickness reduction, and the service life of the coating type refers to a time limit or time range beyond which the coating type is not recommended for use or the coating type is not usable for application, wherein the estimated service life is obtained based on at least one of (i) a change in surface roughness of the coating type, (ii) a change in thickness of the coating type, (iii) gloss, (iv) Griffith's stress, and (v) a wetting angle of the coating type.

**2.** The processor implemented method as claimed in claim 1, further comprising determining a change in surface roughness of the coating type with reference to time based on the second set of chemical-physical correlations of constituents comprised in the coating type.

**3.** The processor implemented method as claimed in claim 2, further comprising determining a change in thickness of the coating type with reference to time based on the first set of chemical-physical correlations and the second set of chemical-physical correlations of constituents comprised in the coating type.

**4.** The processor implemented method as claimed in claim 3, further comprising determining a change in (i) gloss, (ii) a Griffith's stress, and (iii) a wetting angle of the coating type based on the change in surface roughness of the coating type with reference to time.

**5.** A system (100), for predicting properties of a coating type and estimating service life, the system comprising:

a memory (102) storing instructions;
one or more communication interfaces (106); and
one or more hardware processors (104) coupled to the memory (102) via the one or more communication interfaces (106), wherein the one or more hardware processors (104) are configured by the instructions to:

obtain a coating type, and one or more weathering conditions that the coating type is to be subjected in an environment;
identify a polymer for the one or more weathering conditions for the coating type, ;
identify one or more relevant reactions based on the identified polymer and the one or more weathering conditions, wherein identifying the one or more relevant reactions comprises at least one of: identifying initial concentrations of the one or more reactions, kinetic parameters of the one or more reactions, one or more system parameters, reaction constants, solubility and diffusion of oxygen in coating type, spectral sensitivity of light absorbing component;
estimate a mass balance equation for each chemical component comprised in the one or more relevant reactions; and
solve the mass balance equation for each chemical component to (i) predict one or more properties of the coating type, and (ii) obtain an estimated service life of the coating type, wherein the mass balance equation for each chemical component is solved by:

processing the mass balance equation for each chemical component by a first Partial Difference Equations (PDE) solver to obtain (i) a first concentration profile, and (ii) a second concentration profile comprising an associated depth for each chemical component;
estimating, by using a first chemical-physical correlator, a first set of chemical-physical correlations of constituents comprised in the coating type based on (i) the second concentration profile comprising the associated depth and (ii) time at which the coating type is applied and (iii) the one or more weathering conditions that the coating type is to be subjected in the environment;
estimating, via an optimizer, by using (i) the second concentration profile comprising the associated depth and (ii) the time at which the coating type is applied and (iii) the one or more weathering conditions that the coating type is to be subjected in the environment, one or more optimized reactions-based parameters;
processing the one or more optimized reactions-based parameters via a second PDE solver and a third PDE solver to obtain a third concentration profile, and a fourth concentration profile, respectively; and
estimating, by using a second chemical-physical correlator, a second set of chemical-physical correlations of constituents comprised in the coating type using at least one of the third concentration profile, and the fourth concentration profile, wherein

the service life of the coating type is estimated by using the one or more concentration profiles, by predicting changes in physical characteristics of the coating type due to changes in the chemical components in presence of the one or more weather conditions in full 3-D domain, wherein the changes comprises at least

one of non-uniformity in coating degradation, surface roughness, thickness reduction, and the service life of the coating type refers to a time limit or time range beyond which the coating type is not recommended for use or the coating type is not usable for application, wherein the estimated service life is obtained based on at least one of (i) a change in surface roughness of the coating type, (ii) a change in thickness of the coating type, (iii) gloss, (iv) Griffith's stress, and (v) a wetting angle of the coating type.

**6.** The system as claimed in claim 5, wherein the one or more hardware processors are further configured by the instruction to determine a change in surface roughness of the coating type with reference to time based on the second set of chemical-physical correlations of constituents comprised in the coating type.

**7.** The system as claimed in claim 5, wherein the one or more hardware processors are further configured by the instruction to determine a change in thickness of the coating type with reference to time based on the first set of chemical-physical correlations and the second set of chemical-physical correlations of constituents comprised in the coating type.

**8.** The system as claimed in claim 7, wherein the one or more hardware processors are further configured by the instruction to determine a change in (i) gloss, (ii) a Griffith's stress, and (iii) a wetting angle of the coating type based on the change in surface roughness of the coating type with reference to time.

**9.** One or more non-transitory machine-readable information storage mediums for predicting properties of a coating type and estimating service life, comprising one or more instructions which when executed by one or more hardware processors cause:

obtaining, a coating type, and one or more weathering conditions that the coating type is to be subjected in an environment;
identifying a polymer for the one or more weathering conditions for the coating type;
identifying one or more relevant reactions based on the identified polymer and the one or more weathering conditions;
estimating a mass balance equation for each chemical component comprised in the one or more relevant reactions; and
solving the mass balance equation for each chemical component to (i) predict one or more properties of the coating type, and (ii) obtain an estimated service life of the coating type, wherein the step of solving the mass balance equation for each chemical component comprises:

processing the mass balance equation for each chemical component by a first Partial Difference Equations (PDE) solver to obtain (i) a first concentration profile, and (ii) a second concentration profile comprising an associated depth for each chemical component;
estimating, by using a first chemical-physical correlator, a first set of chemical-physical correlations of constituents comprised in the coating type based on (i) the second concentration profile comprising the associated depth and (ii) time at which the coating type is applied and (iii) the one or more weathering conditions that the coating type is to be subjected in the environment;
estimating, via an optimizer, by using (i) the second concentration profile comprising the associated depth and (ii) the time at which the coating type is applied and (iii) the one or more weathering conditions that the coating type is to be subjected in the environment, one or more optimized reactions-based parameters;
processing the one or more optimized reactions-based parameters via a second PDE solver and a third PDE solver to obtain a third concentration profile, and a fourth concentration profile, respectively; and
estimating, by using a second chemical-physical correlator, a second set of chemical-physical correlations of constituents comprised in the coating type using at least one of the third concentration profile, and the fourth concentration profile, wherein

the service life of the coating type is estimated by using the one or more concentration profiles, by predicting changes in physical characteristics of the coating type due to changes in the chemical components in presence of the one or more weather conditions in full 3-D domain, wherein the changes comprises at least one of non-uniformity in coating degradation, surface roughness, thickness reduction, and the service life of the coating type refers to a time limit or time range beyond which the coating type is not recommended for use or the coating type is not usable for application, wherein the estimated service life is obtained based on at least one of (i) a change in surface roughness of the coating type, (ii) a change in thickness of the coating type, (iii) gloss, (iv) Griffith's stress, and (v) a wetting angle of the coating type.

**10.** The one or more non-transitory machine-readable information storage mediums as claimed in claim 9, wherein the one or more instructions which when executed by the one or more hardware processors further cause one or more of:

determining a change in surface roughness of the coating type with reference to time based on the second set of chemical-physical correlations of constituents comprised in the coating type;
determining a change in thickness of the coating type with reference to time based on the first set of chemical-physical correlations and the second set of chemical-physical correlations of constituents comprised in the coating type; and
determining a change in (i) gloss, (ii) a Griffith's stress, and (iii) a wetting angle of the coating type based on the change in surface roughness of the coating type with reference to time.

**Patentansprüche**

**1.** Prozessorimplementiertes Verfahren zum Vorhersagen von Eigenschaften eines Beschichtungstyps und zum Schätzen der Lebensdauer, wobei das prozessorimplementierte Verfahren Folgendes umfasst:

Erhalten, über einen oder mehrere Hardwareprozessoren, eines Beschichtungstyps und einer oder mehrerer Witterungsbedingungen, denen der Beschichtungstyp in einer Umgebung ausgesetzt werden soll (202);
Identifizieren, über den einen oder die mehreren Hardwareprozessoren, eines Polymers für die eine oder die mehreren Witterungsbedingungen für den Beschichtungstyp (204);
Identifizieren, über den einen oder die mehreren Hardwareprozessoren, einer oder mehrerer relevanter Reaktionen basierend auf dem identifizierten Polymer und der einen oder den mehreren Witterungsbedingungen (206), wobei das Identifizieren der einen oder der mehreren relevanten Reaktionen mindestens eines von Folgendem umfasst: Ermittlung der Anfangskonzentrationen der einen oder mehreren Reaktionen, der kinetischen Parameter der einen oder mehreren Reaktionen, der einen oder mehreren Systemparameter, der Reaktionskonstanten, der Löslichkeit und der Fusion von Sauerstoff in der Beschichtungsart, der spektralen Empfindlichkeit der lichtabsorbierenden Komponente;
Schätzen, über den einen oder die mehreren Hardwareprozessoren, einer Massenbilanzgleichung für jede chemische Komponente, die in der einen oder den mehreren relevanten Reaktionen enthalten ist (208); und
Lösen, über den einen oder die mehreren Hardwareprozessoren, der Massenbilanzgleichung für jede chemische Komponente, um (i) eine oder mehrere Eigenschaften des Beschichtungstyps vorherzusagen und (ii) eine geschätzte Lebensdauer des Beschichtungstyps zu erhalten (210), wobei der Schritt des Lösens der Massenbilanzgleichung für jede chemische Komponente Folgendes umfasst:

Erzeugen eines Konzentrationsprofils mit einer Tiefe des Beschichtungstyps;
Empfangen des erzeugten Konzentrationsprofils durch Lösungsmittel für partielle Differenzgleichungen (PDE) zum Abgleichen der kinetischen Parameter und des einen oder der mehreren Systemparameter;
Optimieren des einen oder der mehreren Systemparameter für das erzeugte Konzentrationsprofil;
Senden des optimierten einen oder der optimierten mehreren Systemparameter an das erste Lösungsmittel für partielle Differenzgleichungen (PDE), um eine Dickenreduzierung mit der Zeit, eine Änderung des Konzentrationsprofils mit der Zeit, einen Glanzverlust und einen Massenverlust zu erhalten, wobei der eine oder die mehreren Systemparameter verarbeitet werden, um Fehler bei der Modellierung durch Anpassen des einen oder der mehreren Systemparameter zu minimieren;
Verarbeiten der Massenbilanzgleichung für jede chemische Komponente durch ein erstes Lösungsmittel für partielle Differenzgleichungen (PDE), um (i) ein erstes Konzentrationsprofil und (ii) ein zweites Konzentrationsprofil, das eine zugehörige Tiefe für jede chemische Komponente umfasst, zu erhalten;
Schätzen, unter Verwendung eines ersten chemischphysikalischen Korrelators, eines ersten Satzes chemisch-physikalischer Korrelationen von Bestandteilen, die in dem Beschichtungstyp enthalten sind, basierend auf (i) dem zweiten Konzentrationsprofil, das die zugehörige Tiefe umfasst, und (ii) der Zeit, zu der der Beschichtungstyp aufgetragen wird, und (iii) der einen oder den mehreren Witterungsbedingungen, denen der Beschichtungstyp in der Umgebung ausgesetzt werden soll;
Schätzen, über einen Optimierer, unter Verwendung (i) des zweiten Konzentrationsprofils, das die zugehörige Tiefe umfasst, und (ii) der Zeit, zu der der Beschichtungstyp aufgetragen wird, und (iii) der einen oder der mehreren Witterungsbedingungen, denen der Beschichtungstyp in der Umgebung ausgesetzt werden soll, eines oder mehrerer optimierter reaktionsbasierter Parameter;
Verarbeiten des einen oder der mehreren optimierten reaktionsbasierten Parameter über ein zweites PDE-Lösungsmittel und ein drittes PDE-Lösungsmittel, um ein drittes Konzentrationsprofil bzw. ein viertes

Konzentrationsprofil zu erhalten; und
Schätzen, unter Verwendung eines zweiten chemischphysikalischen Korrelators, eines zweiten Satzes chemisch-physikalischer Korrelationen von Bestandteilen, die in dem Beschichtungstyp enthalten sind, unter Verwendung des dritten Konzentrationsprofils und/oder des vierten Konzentrationsprofils, wobei

die Lebensdauer des Beschichtungstyps unter Verwendung des einen oder der mehreren Konzentrationsprofile geschätzt wird, indem Änderungen der physikalischen Eigenschaften des Beschichtungstyps aufgrund von Änderungen der chemischen Komponenten in Gegenwart der einen oder der mehreren Witterungsbedingungen vorhergesagt werden, wobei die Änderungen die Ungleichmäßigkeit der Beschichtungsverschlechterung und/oder die Oberflächenrauigkeit und/oder die Dickenverringerung umfassen und sich die Lebensdauer des Beschichtungstyps auf eine Zeitgrenze oder einen Zeitbereich bezieht, über die bzw. den hinaus der Beschichtungstyp nicht zur Verwendung empfohlen wird oder der Beschichtungstyp nicht zur Anwendung verwendbar ist, wobei die geschätzte Lebensdauer basierend auf (i) einer Änderung der Oberflächenrauigkeit des Beschichtungstyps und/oder (ii) einer Änderung der Dicke des Beschichtungstyps und/oder (iii) Glanz und/oder (iv) Griffiths Spannung und/oder (v) einem Benetzungswinkel des Beschichtungstyps erhalten wird.

**2.** Prozessorimplementiertes Verfahren nach Anspruch 1, ferner umfassend das Bestimmen einer Änderung der Oberflächenrauigkeit des Beschichtungstyps in Bezug auf die Zeit basierend auf dem zweiten Satz chemisch-physikalischer Korrelationen von Bestandteilen, die in dem Beschichtungstyp enthalten sind.

**3.** Prozessorimplementiertes Verfahren nach Anspruch 2, ferner umfassend das Bestimmen einer Änderung der Dicke des Beschichtungstyps in Bezug auf die Zeit basierend auf dem ersten Satz chemisch-physikalischer Korrelationen und dem zweiten Satz chemisch-physikalischer Korrelationen von Bestandteilen, die in dem Beschichtungstyp enthalten sind.

**4.** Prozessorimplementiertes Verfahren nach Anspruch 3, ferner umfassend das Bestimmen einer Änderung von (i) Glanz, (ii) einer Griffiths Spannung und (iii) eines Benetzungswinkels des Beschichtungstyps basierend auf der Änderung der Oberflächenrauigkeit des Beschichtungstyps in Bezug auf die Zeit.

**5.** System (100) zum Vorhersagen von Eigenschaften eines Beschichtungstyps und zum Schätzen der Lebensdauer, wobei das System Folgendes umfasst:

einen Speicher (102), der Anweisungen speichert;
eine oder mehrere Kommunikationsschnittstellen (106); und
einen oder mehrere Hardwareprozessoren (104), die über die eine oder die mehreren Kommunikationsschnittstellen (106) mit dem Speicher (102) gekoppelt sind, wobei der eine oder die mehreren Hardwareprozessoren (104) durch die Anweisungen konfiguriert sind zum:

Erhalten eines Beschichtungstyps und einer oder mehrerer Witterungsbedingungen, denen der Beschichtungstyp in einer Umgebung ausgesetzt werden soll;
Identifizieren eines Polymers für die eine oder die mehreren Witterungsbedingungen für den Beschichtungstyp;
Identifizieren einer oder mehrerer relevanter Reaktionen basierend auf dem identifizierten Polymer und der einen oder den mehreren Witterungsbedingungen, wobei das Identifizieren der einen oder der mehreren relevanten Reaktionen mindestens eines von Folgendem umfasst: Ermittlung der Anfangskonzentrationen der einen oder mehreren Reaktionen, der kinetischen Parameter der einen oder mehreren Reaktionen, der einen oder mehreren Systemparameter, der Reaktionskonstanten, der Löslichkeit und der Fusion von Sauerstoff in der Beschichtungsart, der spektralen Empfindlichkeit der lichtabsorbierenden Komponente;
Schätzen einer Massenbilanzgleichung für jede chemische Komponente, die in der einen oder den mehreren relevanten Reaktionen enthalten ist; und
Lösen der Massenbilanzgleichung für jede chemische Komponente, um (i) eine oder mehrere Eigenschaften des Beschichtungstyps vorherzusagen und (ii) eine geschätzte Lebensdauer des Beschichtungstyps zu erhalten, wobei die Massenbilanzgleichung für jede chemische Komponente gelöst wird durch:

Verarbeiten der Massenbilanzgleichung für jede chemische Komponente durch ein erstes Lösungsmittel für partielle Differenzgleichungen (PDE), um (i) ein erstes Konzentrationsprofil und (ii) ein zweites Konzentrationsprofil, das eine zugehörige Tiefe für jede chemische Komponente umfasst, zu erhalten;
Schätzen, unter Verwendung eines ersten chemischphysikalischen Korrelators, eines ersten Satzes

chemisch-physikalischer Korrelationen von Bestandteilen, die in dem Beschichtungstyp enthalten sind, basierend auf (i) dem zweiten Konzentrationsprofil, das die zugehörige Tiefe umfasst, und (ii) der Zeit, zu der der Beschichtungstyp aufgetragen wird, und (iii) der einen oder den mehreren Witterungsbedingungen, denen der Beschichtungstyp in der Umgebung ausgesetzt werden soll;
Schätzen, über einen Optimierer, unter Verwendung (i) des zweiten Konzentrationsprofils, das die zugehörige Tiefe umfasst, und (ii) der Zeit, zu der der Beschichtungstyp aufgetragen wird, und (iii) der einen oder der mehreren Witterungsbedingungen, denen der Beschichtungstyp in der Umgebung ausgesetzt werden soll, eines oder mehrerer optimierter reaktionsbasierter Parameter;
Verarbeiten des einen oder der mehreren optimierten reaktionsbasierten Parameter über ein zweites PDE-Lösungsmittel und ein drittes PDE-Lösungsmittel, um ein drittes Konzentrationsprofil bzw. ein viertes Konzentrationsprofil zu erhalten; und
Schätzen, unter Verwendung eines zweiten chemischphysikalischen Korrelators, eines zweiten Satzes chemisch-physikalischer Korrelationen von Bestandteilen, die in dem Beschichtungstyp enthalten sind, unter Verwendung des dritten Konzentrationsprofils und/oder des vierten Konzentrationsprofils, wobei

die Lebensdauer des Beschichtungstyps unter Verwendung des einen oder der mehreren Konzentrationsprofile geschätzt wird, indem Änderungen der physikalischen Eigenschaften des Beschichtungstyps aufgrund von Änderungen der chemischen Komponenten in Gegenwart der einen oder der mehreren Witterungsbedingungen im vollständigen 3-D-Bereich vorhergesagt werden, wobei die Änderungen die Ungleichmäßigkeit der Beschichtungsverschlechterung und/oder die Oberflächenrauigkeit und/oder die Dickenverringerung umfassen und sich die Lebensdauer des Beschichtungstyps auf eine Zeitgrenze oder einen Zeitbereich bezieht, über die bzw. den hinaus der Beschichtungstyp nicht zur Verwendung empfohlen wird oder der Beschichtungstyp nicht zur Anwendung verwendbar ist, wobei die geschätzte Lebensdauer basierend auf (i) einer Änderung der Oberflächenrauigkeit des Beschichtungstyps und/oder (ii) einer Änderung der Dicke des Beschichtungstyps und/oder (iii) Glanz und/oder (iv) Griffiths Spannung und/oder (v) einem Benetzungswinkel des Beschichtungstyps erhalten wird.

**6.** System nach Anspruch 5, wobei der eine oder die mehreren Hardwareprozessoren ferner durch die Anweisung konfiguriert sind, eine Änderung der Oberflächenrauigkeit des Beschichtungstyps in Bezug auf die Zeit basierend auf dem zweiten Satz chemisch-physikalischer Korrelationen von Bestandteilen, die in dem Beschichtungstyp enthalten sind, zu bestimmen.

**7.** System nach Anspruch 5, wobei der eine oder die mehreren Hardwareprozessoren ferner durch die Anweisung konfiguriert sind, eine Änderung der Dicke des Beschichtungstyps in Bezug auf die Zeit basierend auf dem ersten Satz chemisch-physikalischer Korrelationen und dem zweiten Satz chemisch-physikalischer Korrelationen von Bestandteilen, die in dem Beschichtungstyp enthalten sind, zu bestimmen.

**8.** System nach Anspruch 7, wobei der eine oder die mehreren Hardwareprozessoren ferner durch die Anweisung konfiguriert sind, eine Änderung von (i) Glanz, (ii) einer Griffiths Spannung und (iii) eines Benetzungswinkels des Beschichtungstyps basierend auf der Änderung der Oberflächenrauigkeit des Beschichtungstyps in Bezug auf die Zeit zu bestimmen.

**9.** Ein oder mehrere nichtflüchtige maschinenlesbare Informationsspeichermedien zum Vorhersagen von Eigenschaften eines Beschichtungstyps und zum Schätzen der Lebensdauer, umfassend eine oder mehrere Anweisungen, die, wenn sie von einem oder mehreren Hardwareprozessoren ausgeführt werden, Folgendes bewirken:

Erhalten eines Beschichtungstyps und einer oder mehrerer Witterungsbedingungen, denen der Beschichtungstyp in einer Umgebung ausgesetzt werden soll;
Identifizieren eines Polymers für die eine oder die mehreren Witterungsbedingungen für den Beschichtungstyp;
Identifizieren einer oder mehrerer relevanter Reaktionen basierend auf dem identifizierten Polymer und der einen oder den mehreren Witterungsbedingungen;
Schätzen einer Massenbilanzgleichung für jede chemische Komponente, die in der einen oder den mehreren relevanten Reaktionen enthalten ist; und
Lösen der Massenbilanzgleichung für jede chemische Komponente, um (i) eine oder mehrere Eigenschaften des Beschichtungstyps vorherzusagen und (ii) eine geschätzte Lebensdauer des Beschichtungstyps zu erhalten, wobei der Schritt des Lösens der Massenbilanzgleichung für jede chemische Komponente Folgendes umfasst:

Verarbeiten der Massenbilanzgleichung für jede chemische Komponente durch ein erstes Lösungsmittel für

partielle Differenzgleichungen (PDE), um (i) ein erstes Konzentrationsprofil und (ii) ein zweites Konzentrationsprofil, das eine zugehörige Tiefe für jede chemische Komponente umfasst, zu erhalten;
Schätzen, unter Verwendung eines ersten chemischphysikalischen Korrelators, eines ersten Satzes chemisch-physikalischer Korrelationen von Bestandteilen, die in dem Beschichtungstyp enthalten sind, basierend auf (i) dem zweiten Konzentrationsprofil, das die zugehörige Tiefe umfasst, und (ii) der Zeit, zu der der Beschichtungstyp aufgetragen wird, und (iii) der einen oder den mehreren Witterungsbedingungen, denen der Beschichtungstyp in der Umgebung ausgesetzt werden soll;
Schätzen, über einen Optimierer, unter Verwendung (i) des zweiten Konzentrationsprofils, das die zugehörige Tiefe umfasst, und (ii) der Zeit, zu der der Beschichtungstyp aufgetragen wird, und (iii) der einen oder der mehreren Witterungsbedingungen, denen der Beschichtungstyp in der Umgebung ausgesetzt werden soll, eines oder mehrerer optimierter reaktionsbasierter Parameter;
Verarbeiten des einen oder der mehreren optimierten reaktionsbasierten Parameter über ein zweites PDE-Lösungsmittel und ein drittes PDE-Lösungsmittel, um ein drittes Konzentrationsprofil bzw. ein viertes Konzentrationsprofil zu erhalten; und
Schätzen, unter Verwendung eines zweiten chemischphysikalischen Korrelators, eines zweiten Satzes chemisch-physikalischer Korrelationen von Bestandteilen, die in dem Beschichtungstyp enthalten sind, unter Verwendung des dritten Konzentrationsprofils und/oder des vierten Konzentrationsprofils, wobei

die Lebensdauer des Beschichtungstyps unter Verwendung des einen oder der mehreren Konzentrationsprofile geschätzt wird, indem Änderungen der physikalischen Eigenschaften des Beschichtungstyps aufgrund von Änderungen der chemischen Komponenten in Gegenwart der einen oder der mehreren Witterungsbedingungen im vollständigen 3-D-Bereich vorhergesagt werden, wobei die Änderungen die Ungleichmäßigkeit der Beschichtungsverschlechterung und/oder die Oberflächenrauigkeit und/oder die Dickenverringerung umfassen und sich die Lebensdauer des Beschichtungstyps auf eine Zeitgrenze oder einen Zeitbereich bezieht, über die bzw. den hinaus der Beschichtungstyp nicht zur Verwendung empfohlen wird oder der Beschichtungstyp nicht zur Anwendung verwendbar ist, wobei die geschätzte Lebensdauer basierend auf (i) einer Änderung der Oberflächenrauigkeit des Beschichtungstyps und/oder (ii) einer Änderung der Dicke des Beschichtungstyps und/oder (iii) Glanz und/oder (iv) Griffiths Spannung und/oder (v) einem Benetzungswinkel des Beschichtungstyps erhalten wird.

**10.** Ein oder mehrere nichtflüchtige maschinenlesbare Informationsspeichermedien nach Anspruch 9, wobei die eine oder die mehreren Anweisungen, die, wenn sie von dem einen oder den mehreren Hardwareprozessoren ausgeführt werden, ferner eines oder mehrere der Folgenden bewirken:

Bestimmen einer Änderung der Oberflächenrauigkeit des Beschichtungstyps in Bezug auf die Zeit basierend auf dem zweiten Satz chemisch-physikalischer Korrelationen von Bestandteilen, die in dem Beschichtungstyp enthalten sind;
Bestimmen einer Änderung der Dicke des Beschichtungstyps in Bezug auf die Zeit basierend auf dem ersten Satz chemisch-physikalischer Korrelationen und dem zweiten Satz chemisch-physikalischer Korrelationen von Bestandteilen, die in dem Beschichtungstyp enthalten sind; und
Bestimmen einer Änderung von (i) Glanz, (ii) einer Griffiths Spannung und (iii) eines Benetzungswinkels des Beschichtungstyps basierend auf der Änderung der Oberflächenrauigkeit des Beschichtungstyps in Bezug auf die Zeit.

## Revendications

**1.** Procédé mis en œuvre par processeur pour prédire des propriétés d'un type de revêtement et estimer une durée de vie utile, le procédé mis en œuvre par processeur comprenant :

l'obtention, par l'intermédiaire d'un ou plusieurs processeurs matériels, d'un type de revêtement, et d'une ou plusieurs conditions de vieillissement auxquelles le type de revêtement doit être soumis dans un environnement (202) ;
l'identification, par l'intermédiaire des un ou plusieurs processeurs matériels, d'un polymère pour les une ou plusieurs conditions de vieillissement pour le type de revêtement (204) ;
l'identification, par l'intermédiaire des un ou plusieurs processeurs matériels, d'une ou plusieurs réactions pertinentes sur la base du polymère identifié et des une ou plusieurs conditions de vieillissement (206), dans lequel l'identification des une ou plusieurs réactions pertinentes comprend au moins l'un parmi : l'identification de

concentrations initiales des une ou plusieurs réactions, de paramètres cinétiques des une ou plusieurs réactions, d'un ou plusieurs paramètres de système, de constantes de réaction, de solubilité et de diffusion d'oxygène dans le type de revêtement, de sensibilité spectrale d'un composant absorbant la lumière ;
l'estimation, par l'intermédiaire des un ou plusieurs processeurs matériels, d'une équation d'équilibre de masse pour chaque composant chimique compris dans les une ou plusieurs réactions pertinentes (208) ; et la résolution, par l'intermédiaire des un ou plusieurs processeurs matériels, de l'équation d'équilibre de masse pour chaque composant chimique pour (i) prédire une ou plusieurs propriétés du type de revêtement, et (ii) obtenir une durée de vie utile estimée du type de revêtement (210), dans lequel l'étape de résolution de l'équation d'équilibre de masse pour chaque composant chimique comprend :

la génération d'un profil de concentration avec une profondeur du type de revêtement ;
la réception du profil de concentration généré par un solveur d'équations de différence partielle (PDE) pour faire correspondre les paramètres cinétiques et les un ou plusieurs paramètres de système ;
l'optimisation des un ou plusieurs paramètres de système pour le profil de concentration généré ;
l'envoi des un ou plusieurs paramètres de système optimisés au premier solveur d'équations de différence partielle (PDE) pour obtenir une réduction d'épaisseur avec le temps, un changement du profil de concentration avec le temps, une perte de brillance, brillant une perte de masse, dans lequel les un ou plusieurs paramètres de système sont traités pour minimiser des erreurs de modélisation en ajustant les un ou plusieurs paramètres de système ;
le traitement de l'équation d'équilibre de masse pour chaque composant chimique par un premier solveur d'équations de différence partielle (PDE) pour obtenir (i) un premier profil de concentration, et (ii) un second profil de concentration comprenant une profondeur associée pour chaque composant chimique ;
l'estimation, en utilisant un premier corrélateur chimique-physique, d'un premier ensemble de corrélations chimique-physique de constituants compris dans le type de revêtement sur la base (i) du second profil de concentration comprenant la profondeur associée et (ii) du temps auquel le type de revêtement est appliqué et (iii) des une ou plusieurs conditions de vieillissement auxquelles le type de revêtement doit être soumis dans l'environnement ;
l'estimation, par l'intermédiaire d'un optimiseur, en utilisant (i) le second profil de concentration comprenant la profondeur associée et (ii) le temps auquel le type de revêtement est appliqué et (iii) les une ou plusieurs conditions de vieillissement auxquelles le type de revêtement doit être soumis dans l'environnement, d'un ou plusieurs paramètres basés sur des réactions optimisées ;
le traitement des un ou plusieurs paramètres basés sur des réactions optimisées par l'intermédiaire d'un deuxième solveur de PDE et d'un troisième solveur de PDE pour obtenir un troisième profil de concentration, et un quatrième profil de concentration, respectivement ; et
l'estimation, en utilisant un second corrélateur chimique-physique, d'un second ensemble de corrélations chimique-physique de constituants compris dans le type de revêtement en utilisant au moins l'un du troisième profil de concentration, et du quatrième profil de concentration, dans lequel la durée de vie utile du type de revêtement est estimée en utilisant les un ou plusieurs profils de concentration, en prédisant des changements de caractéristiques physiques du type de revêtement dus à des changements des composants chimiques en présence des une ou plusieurs conditions de vieillissement, dans lequel les changements comprennent au moins l'un d'une non-uniformité de dégradation de revêtement, d'une rugosité de surface, d'une réduction d'épaisseur, et la durée de vie utile du type de revêtement se réfère à une limite de temps ou une plage de temps au-delà de laquelle le type de revêtement n'est pas recommandé pour une utilisation ou le type de revêtement n'est pas utilisable pour une application, dans lequel la durée de vie utile estimée est obtenue sur la base d'au moins l'un de (i) un changement de rugosité de surface du type de revêtement, (ii) un changement d'épaisseur du type de revêtement, (iii) un brillant, (iv) une contrainte de Griffith, et (v) un angle de mouillage du type de revêtement.

**2.** Procédé mis en œuvre par processeur selon la revendication 1, comprenant en outre la détermination d'un changement de rugosité de surface du type de revêtement en référence au temps sur la base du second ensemble de corrélations chimique-physique de constituants compris dans le type de revêtement.

**3.** Procédé mis en œuvre par processeur selon la revendication 2, comprenant en outre la détermination d'un changement d'épaisseur du type de revêtement en référence au temps sur la base du premier ensemble de corrélations chimique-physique et du second ensemble de corrélations chimique-physique de constituants compris dans le type de revêtement.

**4.** Procédé mis en œuvre par processeur selon la revendication 3, comprenant en outre la détermination d'un

changement de (i) brillant, (ii) une contrainte de Griffith, et (iii) un angle de mouillage du type de revêtement sur la base du changement de rugosité de surface du type de revêtement en référence au temps.

**5.** Système (100), pour prédire des propriétés d'un type de revêtement et estimer une durée de vie utile, le système comprenant :

une mémoire (102) stockant des instructions ;
une ou plusieurs interfaces de communication (106) ; et
un ou plusieurs processeurs matériels (104) couplés à la mémoire (102) par l'intermédiaire des une ou plusieurs interfaces de communication (106), dans lequel les un ou plusieurs processeurs matériels (104) sont configurés par les instructions pour :

obtenir un type de revêtement, et une ou plusieurs conditions de vieillissement auxquelles le type de revêtement doit être soumis dans un environnement ;
identifier un polymère pour les une ou plusieurs conditions de vieillissement pour le type de revêtement ;
identifier une ou plusieurs réactions pertinentes sur la base du polymère identifié et des une ou plusieurs conditions de vieillissement, dans lequel l'identification des une ou plusieurs réactions pertinentes comprend au moins l'un parmi : l'identification de concentrations initiales des une ou plusieurs réactions, de paramètres cinétiques des une ou plusieurs réactions, d'un ou plusieurs paramètres de système, de constantes de réaction, de solubilité et de diffusion d'oxygène dans le type de revêtement, de sensibilité spectrale d'un composant absorbant la lumière ;
estimer une équation d'équilibre de masse pour chaque composant chimique compris dans les une ou plusieurs réactions pertinentes ; et
résoudre l'équation d'équilibre de masse pour chaque composant chimique pour (i) prédire une ou plusieurs propriétés du type de revêtement, et (ii) obtenir une durée de vie utile estimée du type de revêtement, dans lequel l'équation d'équilibre de masse pour chaque composant chimique est résolue par :

le traitement de l'équation d'équilibre de masse pour chaque composant chimique par un premier solveur d'équations de différence partielle (PDE) pour obtenir (i) un premier profil de concentration, et (ii) un second profil de concentration comprenant une profondeur associée pour chaque composant chimique ;
l'estimation, en utilisant un premier corrélateur chimique-physique, d'un premier ensemble de corrélations chimique-physique de constituants compris dans le type de revêtement sur la base (i) du second profil de concentration comprenant la profondeur associée et (ii) du temps auquel le type de revêtement est appliqué et (iii) des une ou plusieurs conditions de vieillissement auxquelles le type de revêtement doit être soumis dans l'environnement ;
l'estimation, par l'intermédiaire d'un optimiseur, en utilisant (i) le second profil de concentration comprenant la profondeur associée et (ii) le temps auquel le type de revêtement est appliqué et (iii) les une ou plusieurs conditions de vieillissement auxquelles le type de revêtement doit être soumis dans l'environnement, d'un ou plusieurs paramètres basés sur des réactions optimisées ;
le traitement des un ou plusieurs paramètres basés sur des réactions optimisées par l'intermédiaire d'un deuxième solveur de PDE et d'un troisième solveur de PDE pour obtenir un troisième profil de concentration, et un quatrième profil de concentration, respectivement ; et
l'estimation, en utilisant un second corrélateur chimique-physique, d'un second ensemble de corrélations chimique-physique de constituants compris dans le type de revêtement en utilisant au moins l'un du troisième profil de concentration, et du quatrième profil de concentration, dans lequel
la durée de vie utile du type de revêtement est estimée en utilisant les un ou plusieurs profils de concentration, en prédisant des changements de caractéristiques physiques du type de revêtement dus à des changements des composants chimiques en présence des une ou plusieurs conditions de vieillissement dans le domaine 3-D complet, dans lequel les changements comprennent au moins l'un d'une non-uniformité de dégradation de revêtement, d'une rugosité de surface, d'une réduction d'épaisseur, et la durée de vie utile du type de revêtement se réfère à une limite de temps ou une plage de temps au-delà de laquelle le type de revêtement n'est pas recommandé pour une utilisation ou le type de revêtement n'est pas utilisable pour une application, dans lequel la durée de vie utile estimée est obtenue sur la base d'au moins l'un de (i) un changement de rugosité de surface du type de revêtement, (ii) un changement d'épaisseur du type de revêtement, (iii) un brillant, (iv) une contrainte de Griffith, et (v) un angle de mouillage du type de revêtement.

6. Système selon la revendication 5, dans lequel les un ou plusieurs processeurs matériels sont en outre configurés par l'instruction pour déterminer un changement de rugosité de surface du type de revêtement en référence au temps sur la base du second ensemble de corrélations chimique-physique de constituants compris dans le type de revêtement.

7. Système selon la revendication 5, dans lequel les un ou plusieurs processeurs matériels sont en outre configurés par l'instruction pour déterminer un changement d'épaisseur du type de revêtement en référence au temps sur la base du premier ensemble de corrélations chimique-physique et du second ensemble de corrélations chimique-physique de constituants compris dans le type de revêtement.

8. Système selon la revendication 7, dans lequel les un ou plusieurs processeurs matériels sont en outre configurés par l'instruction pour déterminer un changement de (i) brillant, (ii) une contrainte de Griffith, et (iii) un angle de mouillage du type de revêtement sur la base du changement de rugosité de surface du type de revêtement en référence au temps.

9. Un ou plusieurs supports de stockage d'informations lisibles par machine non transitoires pour prédire des propriétés d'un type de revêtement et estimer une durée de vie utile, comprenant une ou plusieurs instructions qui, lorsqu'elles sont exécutées par un ou plusieurs processeurs matériels, amènent :

l'obtention, d'un type de revêtement, et d'une ou plusieurs conditions de vieillissement auxquelles le type de revêtement doit être soumis dans un environnement ;
l'identification d'un polymère pour les une ou plusieurs conditions de vieillissement pour le type de revêtement ;
l'identification d'une ou plusieurs réactions pertinentes sur la base du polymère identifié et des une ou plusieurs conditions de vieillissement ;
l'estimation d'une équation d'équilibre de masse pour chaque composant chimique compris dans les une ou plusieurs réactions pertinentes ; et
la résolution de l'équation d'équilibre de masse pour chaque composant chimique pour (i) prédire une ou plusieurs propriétés du type de revêtement, et (ii) obtenir une durée de vie utile estimée du type de revêtement, dans lequel l'étape de résolution de l'équation d'équilibre de masse pour chaque composant chimique comprend :

le traitement de l'équation d'équilibre de masse pour chaque composant chimique par un premier solveur d'équations de différence partielle (PDE) pour obtenir (i) un premier profil de concentration, et (ii) un second profil de concentration comprenant une profondeur associée pour chaque composant chimique ;
l'estimation, en utilisant un premier corrélateur chimique-physique, d'un premier ensemble de corrélations chimique-physique de constituants compris dans le type de revêtement sur la base (i) du second profil de concentration comprenant la profondeur associée et (ii) du temps auquel le type de revêtement est appliqué et (iii) des une ou plusieurs conditions de vieillissement auxquelles le type de revêtement doit être soumis dans l'environnement ;
l'estimation, par l'intermédiaire d'un optimiseur, en utilisant (i) le second profil de concentration comprenant la profondeur associée et (ii) le temps auquel le type de revêtement est appliqué et (iii) les une ou plusieurs conditions de vieillissement auxquelles le type de revêtement doit être soumis dans l'environnement, d'un ou plusieurs paramètres basés sur des réactions optimisées ;
le traitement des un ou plusieurs paramètres basés sur des réactions optimisées par l'intermédiaire d'un deuxième solveur de PDE et d'un troisième solveur de PDE pour obtenir un troisième profil de concentration, et un quatrième profil de concentration, respectivement ; et
l'estimation, en utilisant un second corrélateur chimique-physique, d'un second ensemble de corrélations chimique-physique de constituants compris dans le type de revêtement en utilisant au moins l'un du troisième profil de concentration, et du quatrième profil de concentration, dans lequel
la durée de vie utile du type de revêtement est estimée en utilisant les un ou plusieurs profils de concentration, en prédisant des changements de caractéristiques physiques du type de revêtement dus à des changements des composants chimiques en présence des une ou plusieurs conditions de vieillissement dans le domaine 3-D complet, dans lequel les changements comprennent au moins l'un d'une non-uniformité de dégradation de revêtement, d'une rugosité de surface, d'une réduction d'épaisseur, et la durée de vie utile du type de revêtement se réfère à une limite de temps ou une plage de temps au-delà de laquelle le type de revêtement n'est pas recommandé pour une utilisation ou le type de revêtement n'est pas utilisable pour une application, dans lequel la durée de vie utile estimée est obtenue sur la base d'au moins l'un de (i) un changement de rugosité de surface du type de revêtement, (ii) un changement d'épaisseur du type de revêtement, (iii) un brillant, (iv) une contrainte de Griffith, et (v) un angle de mouillage du type de revêtement.

10. Un ou plusieurs supports de stockage d'informations lisibles par machine non transitoires selon la revendication 9,

dans lequel les une ou plusieurs instructions qui, lorsqu'elles sont exécutées par les un ou plusieurs processeurs matériels, amènent en outre un ou plusieurs de :

la détermination d'un changement de rugosité de surface du type de revêtement en référence au temps sur la base du second ensemble de corrélations chimique-physique de constituants compris dans le type de revêtement ;
la détermination d'un changement d'épaisseur du type de revêtement en référence au temps sur la base du premier ensemble de corrélations chimique-physique et du second ensemble de corrélations chimique-physique de constituants compris dans le type de revêtement ; et
la détermination d'un changement de (i) brillant, (ii) une contrainte de Griffith, et (iii) un angle de mouillage du type de revêtement sur la base du changement de rugosité de surface du type de revêtement en référence au temps.

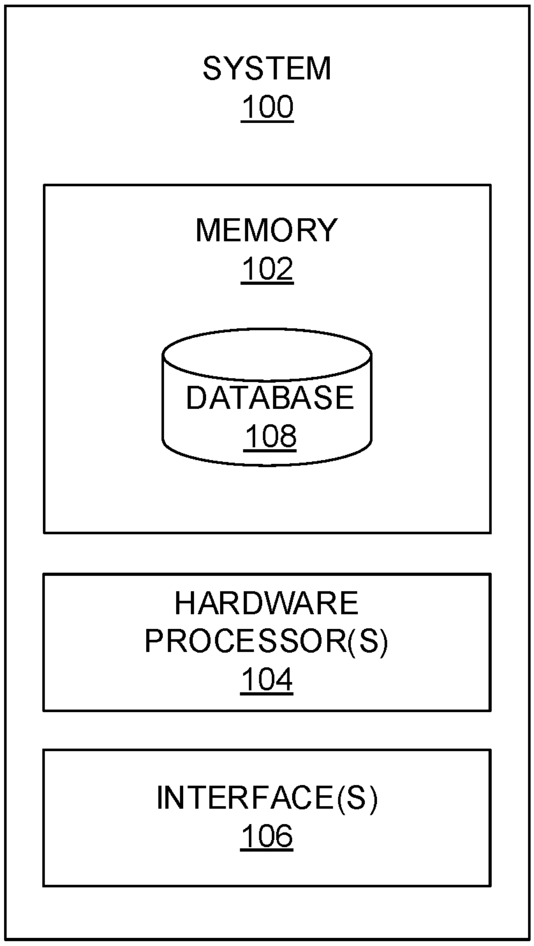
SYSTEM
100
MEMORY
102
DATABASE
108
HARDWARE
PROCESSOR(S)
104
INTERFACE(S)
106

**FIG. 1**

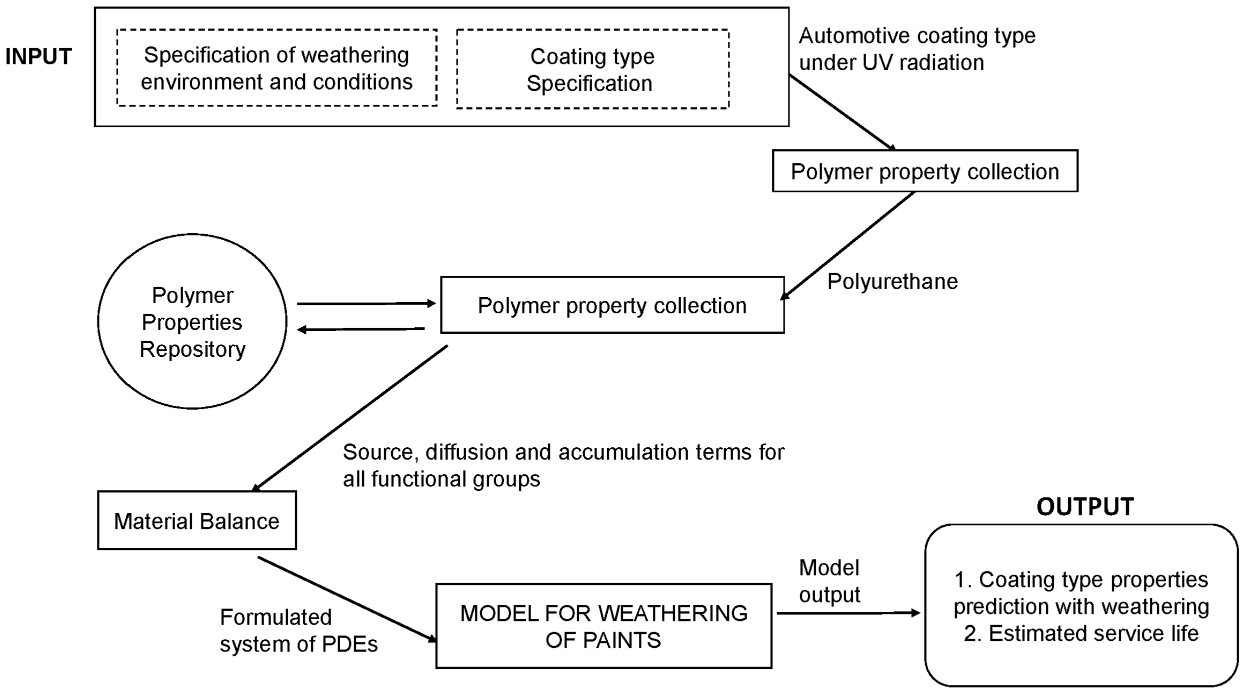
INPUT
Specification of weathering environment and conditions
Coating type Specification
Automotive coating type under UV radiation
Polymer property collection
Polymer Properties Repository
Polymer property collection
Polyurethane
Source, diffusion and accumulation terms for all functional groups
Material Balance
OUTPUT
Formulated system of PDEs
MODEL FOR WEATHERING OF PAINTS
Model output
1. Coating type properties prediction with weathering
2. Estimated service life

FIG. 2

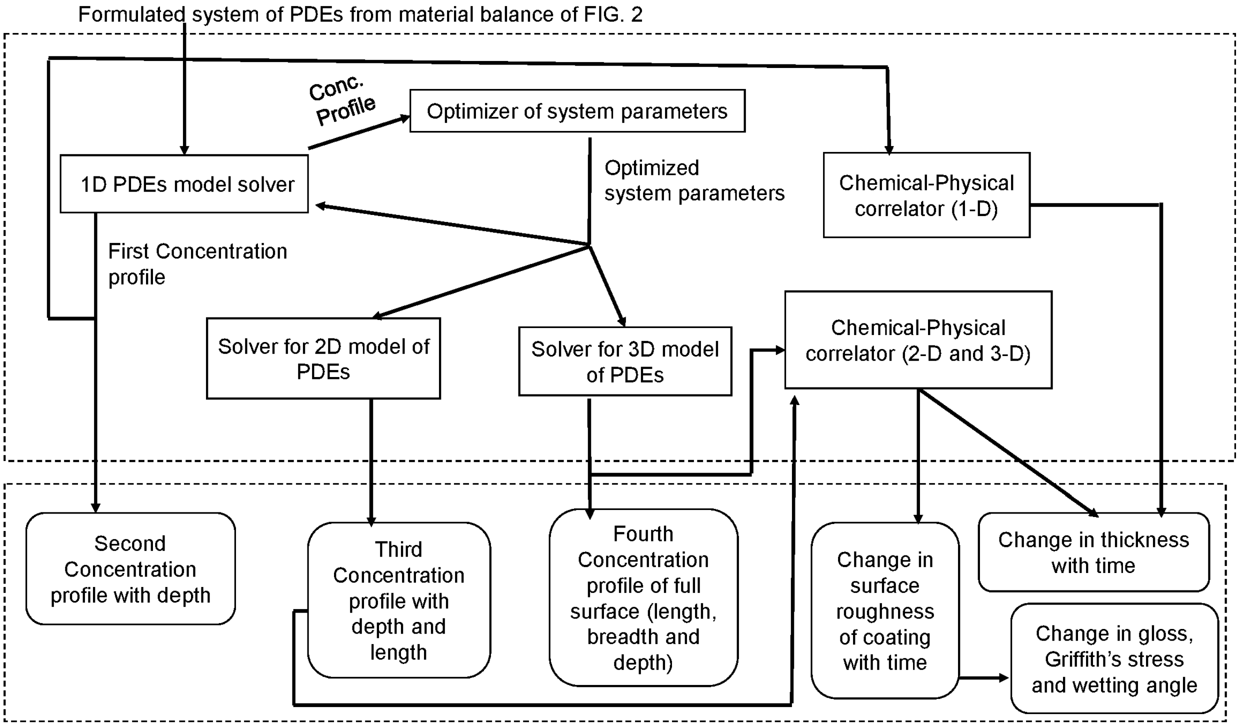

Formulated system of PDEs from material balance of FIG. 2
Conc. Profile
Optimizer of system parameters
1D PDEs model solver
Optimized system parameters
Chemical-Physical correlator (1-D)
First Concentration profile
Solver for 2D model of PDEs
Solver for 3D model of PDEs
Chemical-Physical correlator (2-D and 3-D)
Second Concentration profile with depth
Third Concentration profile with depth and length
Fourth Concentration profile of full surface (length, breadth and depth)
Change in surface roughness of coating with time
Change in thickness with time
Change in gloss, Griffith's stress and wetting angle


FIG. 3

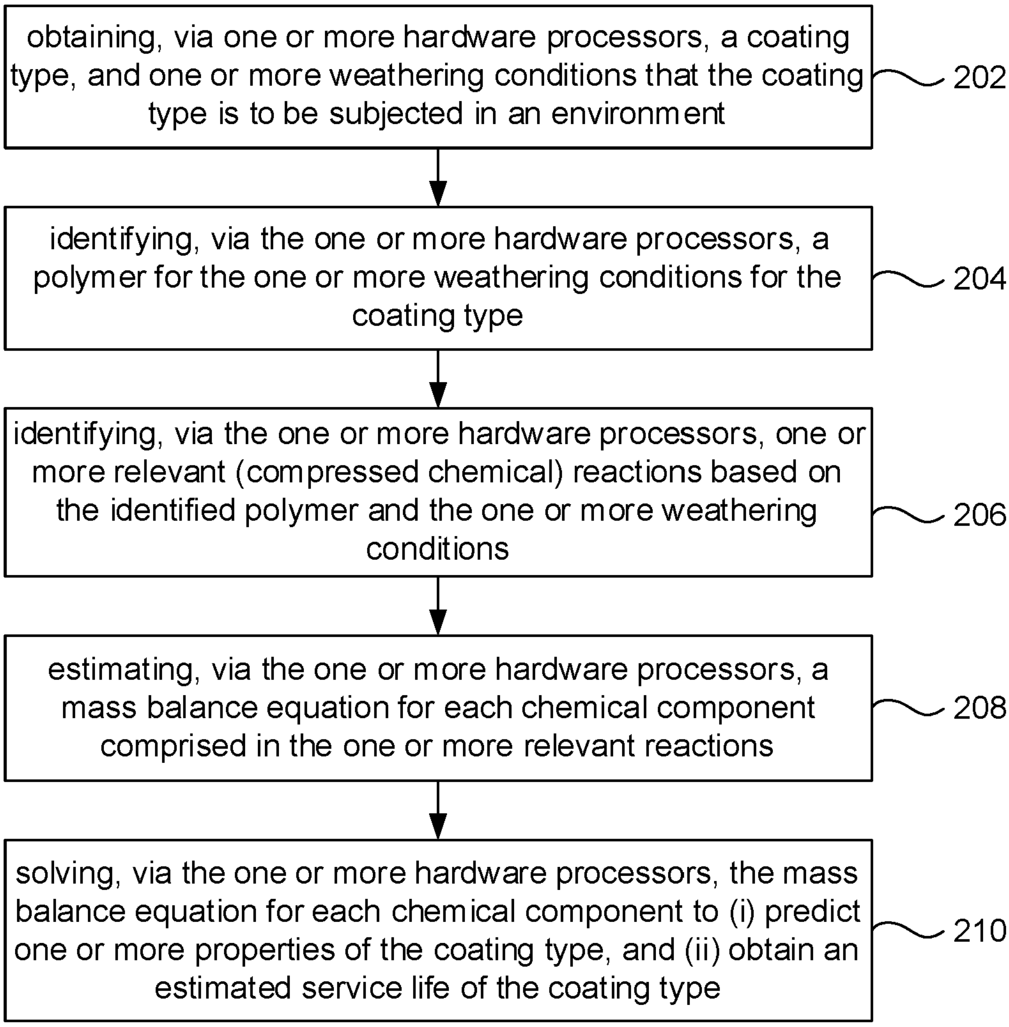
obtaining, via one or more hardware processors, a coating type, and one or more weathering conditions that the coating type is to be subjected in an environment
202
identifying, via the one or more hardware processors, a polymer for the one or more weathering conditions for the coating type
204
identifying, via the one or more hardware processors, one or more relevant (compressed chemical) reactions based on the identified polymer and the one or more weathering conditions
206
estimating, via the one or more hardware processors, a mass balance equation for each chemical component comprised in the one or more relevant reactions
208
solving, via the one or more hardware processors, the mass balance equation for each chemical component to (i) predict one or more properties of the coating type, and (ii) obtain an estimated service life of the coating type
210

FIG. 4

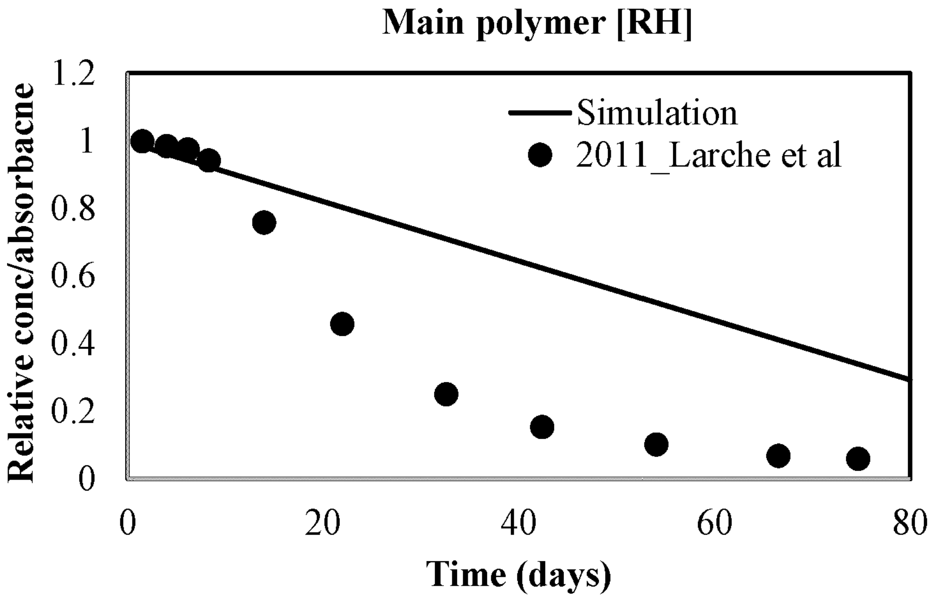
Main polymer [RH]
Relative conc/absorbacne
1.2
1
0.8
0.6
0.4
0.2
0
0
20
40
60
80
Time (days)
Simulation
2011_Larche et al

FIG. 5A

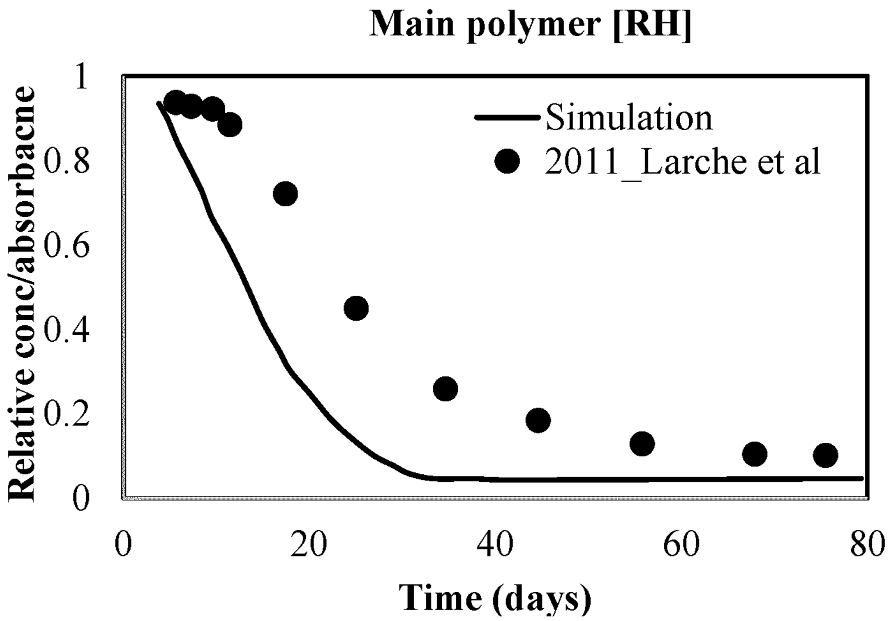
Main polymer [RH]
Relative conc/absorbacne
1
0.8
0.6
0.4
0.2
0
0
20
40
60
80
Time (days)
Simulation
2011_Larche et al

FIG. 5B

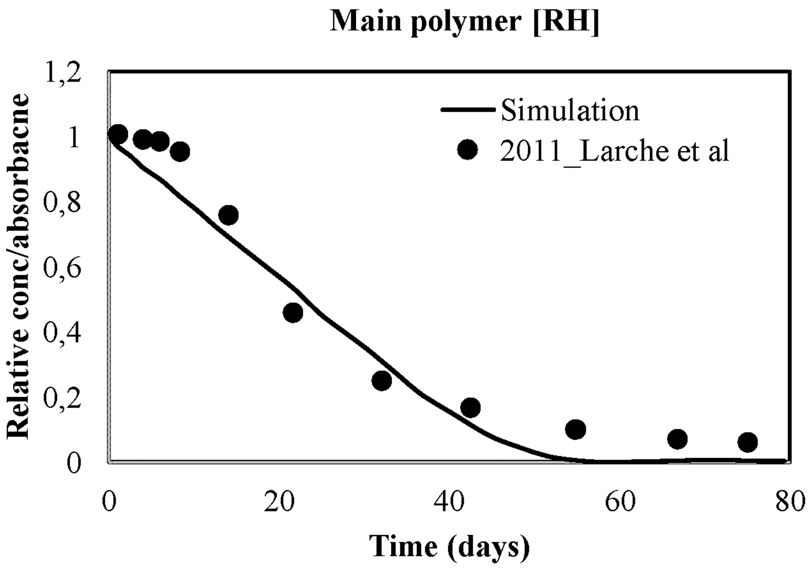
Main polymer [RH]
Relative conc/absorbacne
Time (days)
Simulation
2011_Larche et al
1,2
1
0,8
0,6
0,4
0,2
0
0
20
40
60
80

FIG. 5C

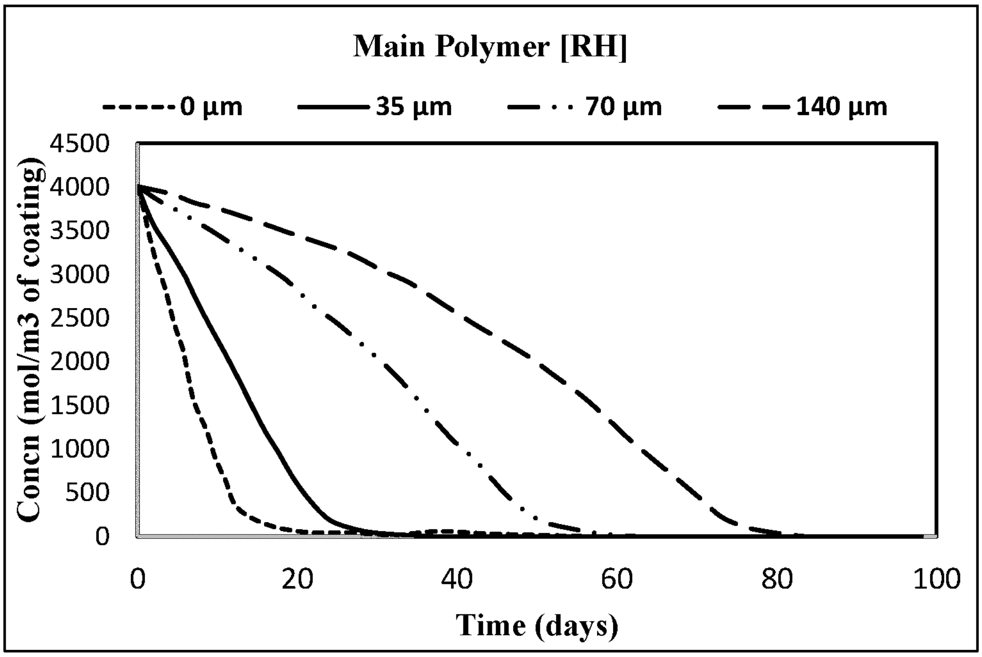
Main Polymer [RH]
0 µm
35 µm
70 µm
140 µm
Concn (mol/m3 of coating)
4500
4000
3500
3000
2500
2000
1500
1000
500
0
0
20
40
60
80
100
Time (days)

FIG. 6A

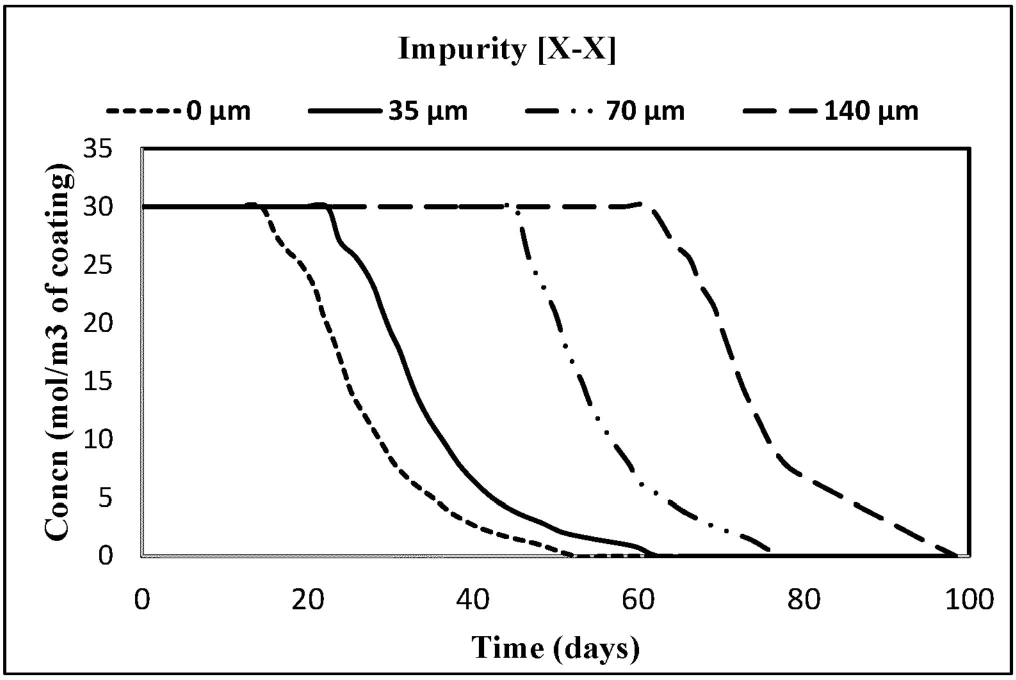
Impurity [X-X]
0 µm
35 µm
70 µm
140 µm
Concn (mol/m3 of coating)
35
30
25
20
15
10
5
0
0
20
40
60
80
100
Time (days)

FIG. 6B

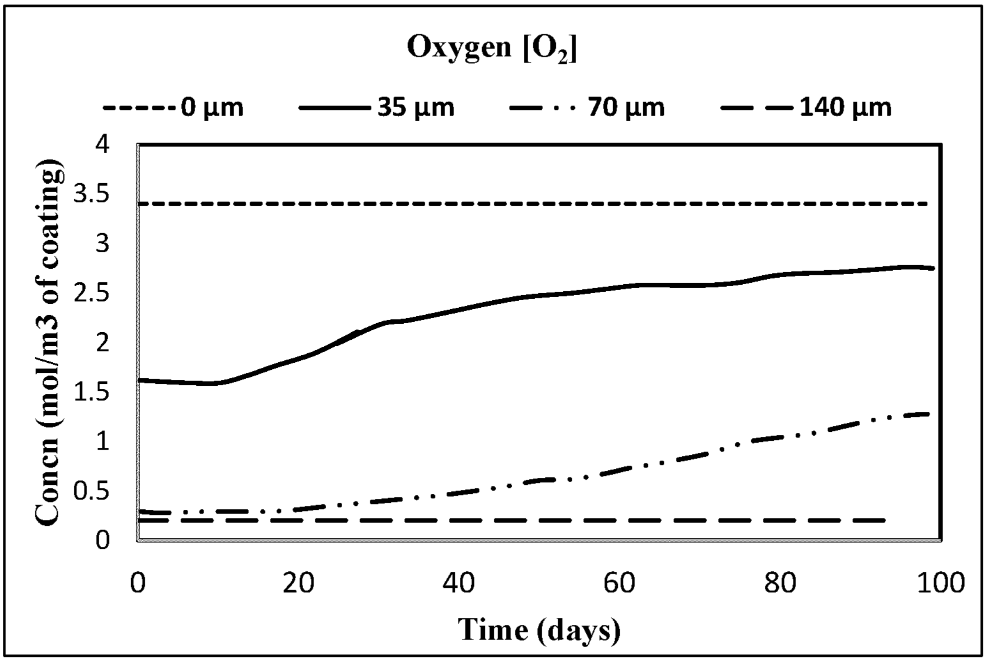
Oxygen [$O_2$]
0 µm
35 µm
70 µm
140 µm
Concn (mol/m3 of coating)
0
0.5
1
1.5
2
2.5
3
3.5
4
0
20
40
60
80
100
Time (days)

FIG. 6C

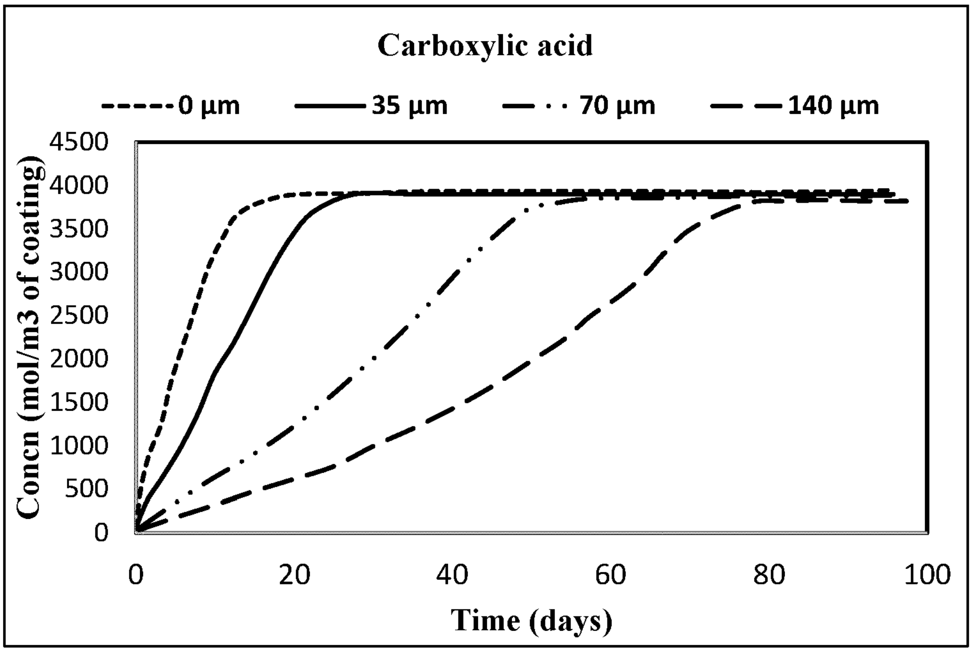
Carboxylic acid
0 µm
35 µm
70 µm
140 µm
Concn (mol/m3 of coating)
0
500
1000
1500
2000
2500
3000
3500
4000
4500
0
20
40
60
80
100
Time (days)

FIG. 6D

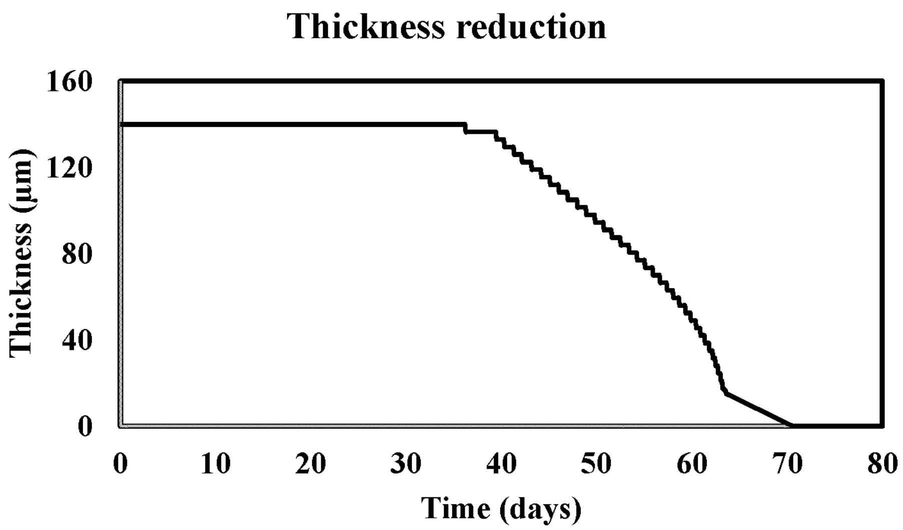
Thickness reduction
160
120
80
40
0
Thickness (µm)
0
10
20
30
40
50
60
70
80
Time (days)

FIG. 7

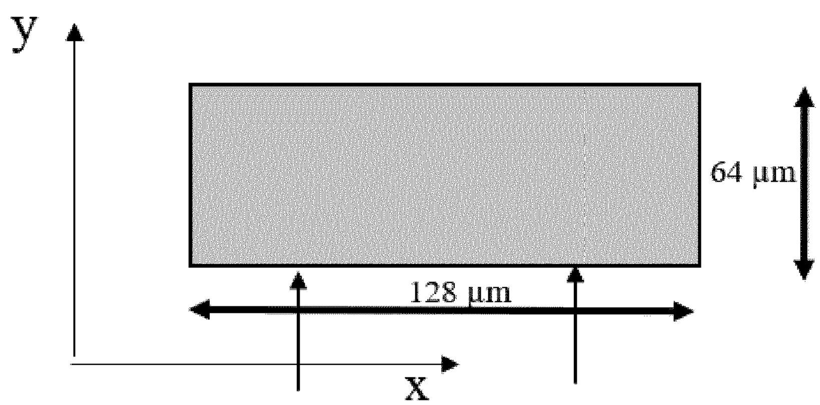
y
64 µm
128 µm
x

FIG. 8

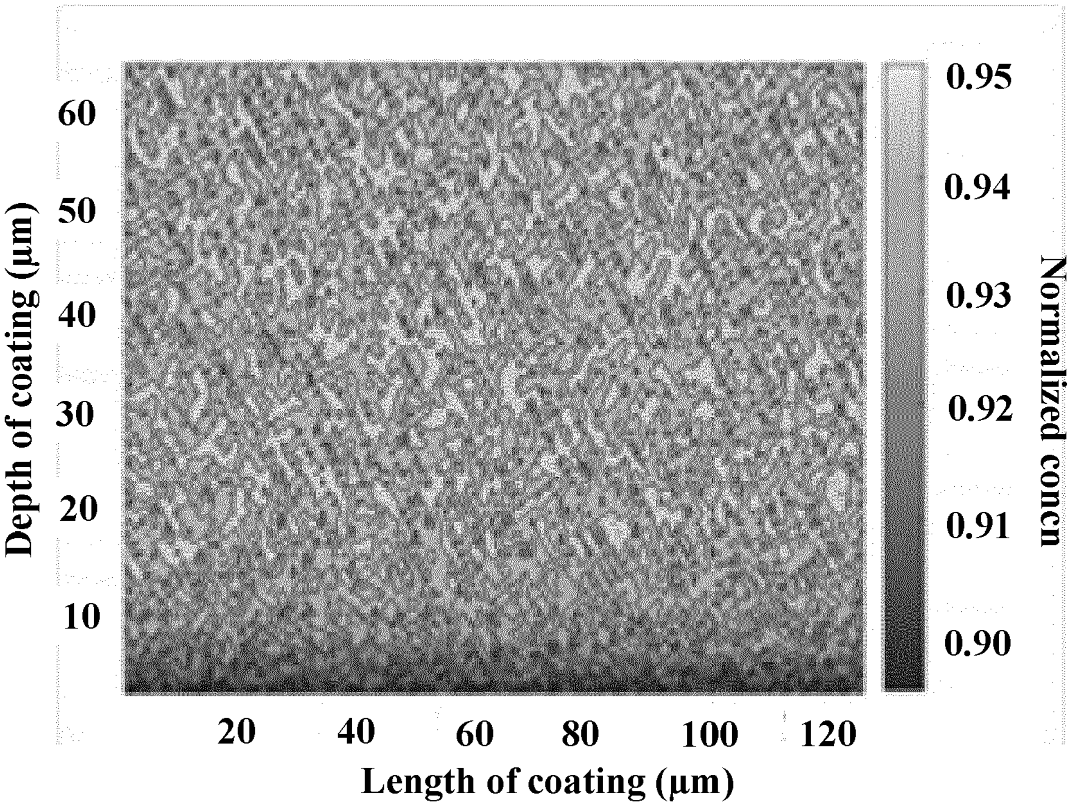
60
50
40
30
20
10
Depth of coating (µm)
20
40
60
80
100
120
Length of coating (µm)
0.95
0.94
0.93
0.92
0.91
0.90
Normalized concn

FIG. 9A

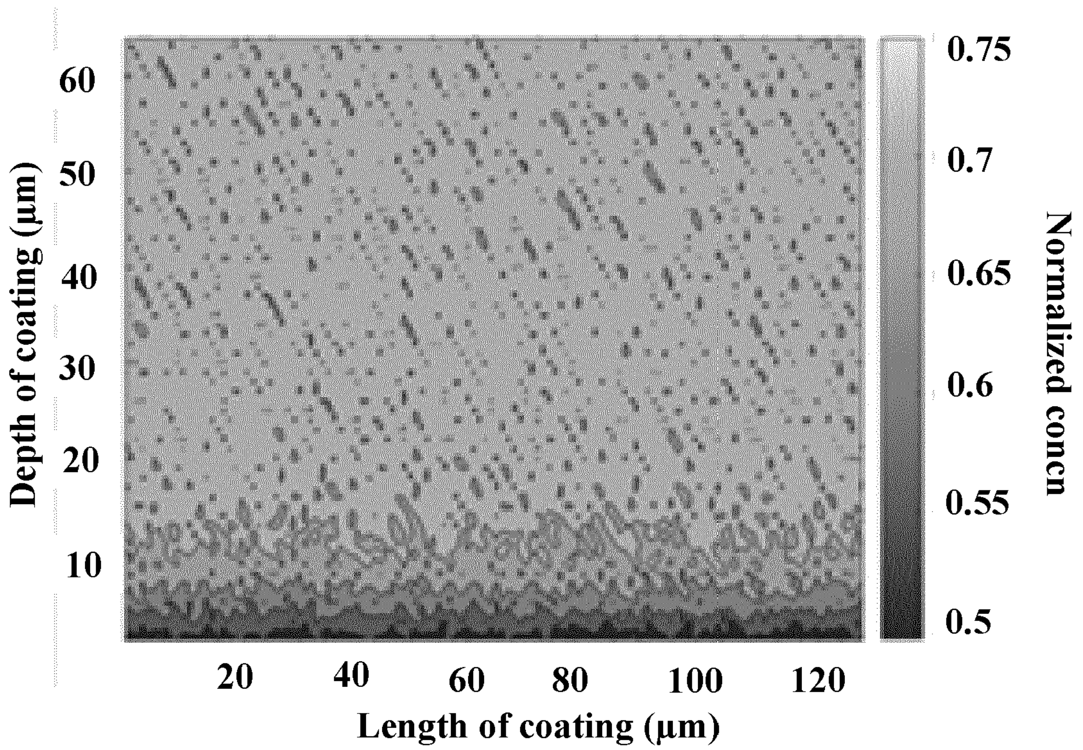
Depth of coating (µm)
60
50
40
30
20
10
20
40
60
80
100
120
Length of coating (µm)
0.75
0.7
0.65
0.6
0.55
0.5
Normalized concn

FIG. 9B

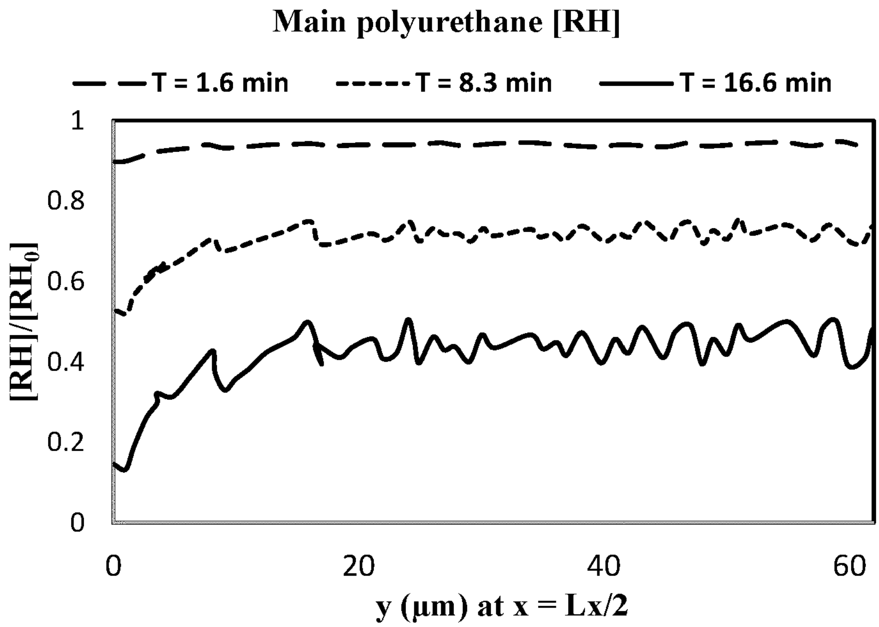


FIG. 10A

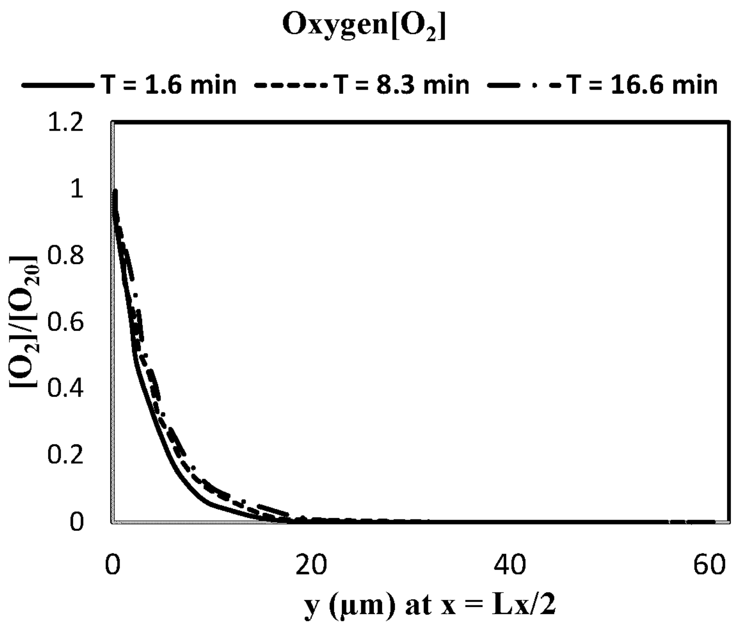


FIG. 10B

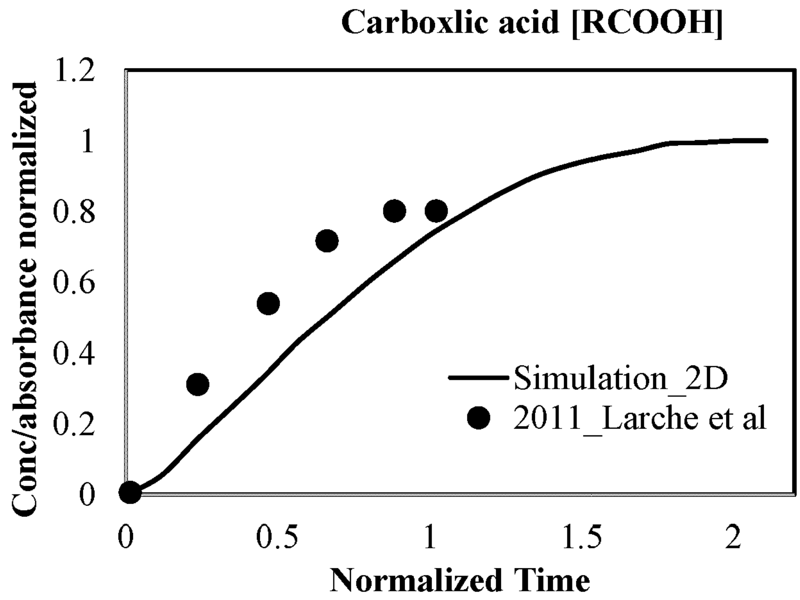
Carboxlic acid [RCOOH]
Conc/absorbance normalized
1.2
1
0.8
0.6
0.4
0.2
0
0
0.5
1
1.5
2
Normalized Time
Simulation_2D
2011_Larche et al

FIG. 11

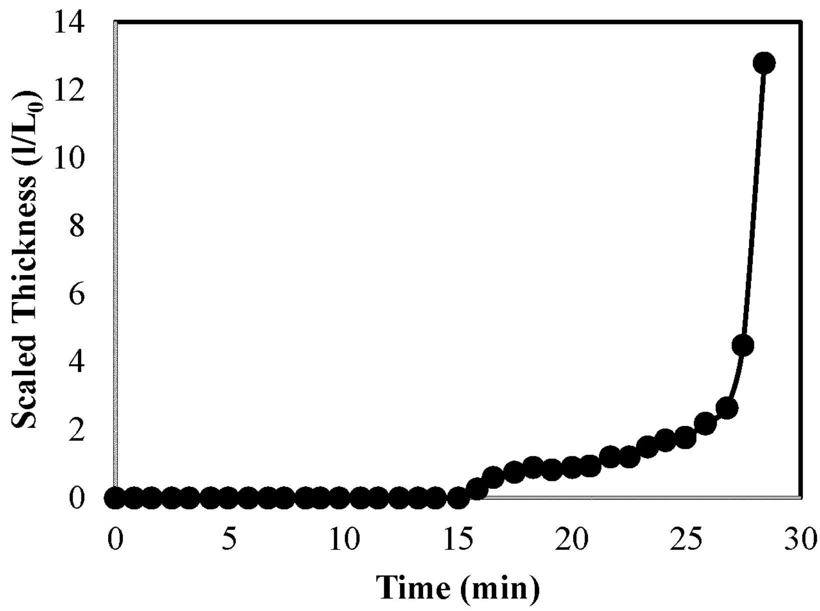
Scaled Thickness (l/L0)
14
12
10
8
6
4
2
0
0
5
10
15
20
25
30
Time (min)

FIG. 12

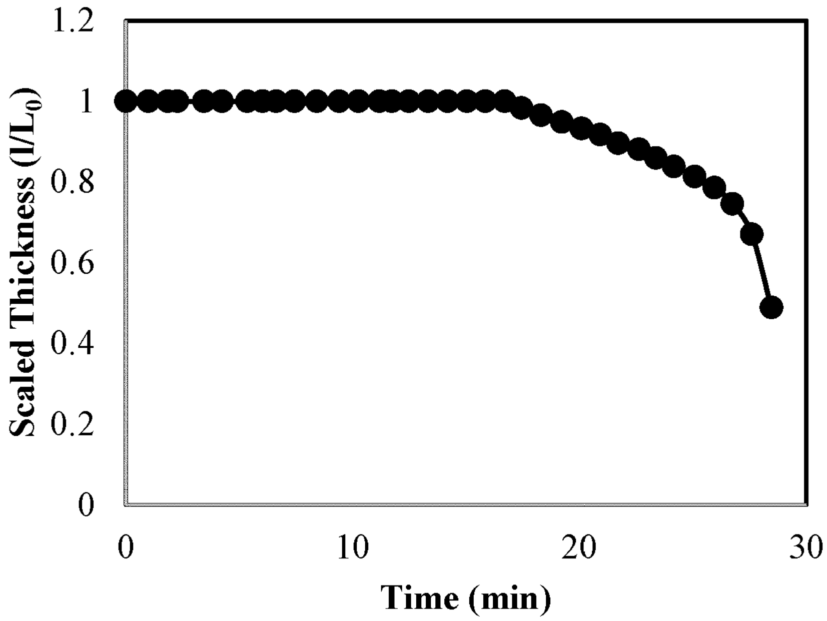


FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- IN 202221064312


### Non-patent literature cited in the description


- **CROLL S. et al.** A framework for predicting the service lifetime of composite polymeric coatings. *JOURNAL OF MATERIAL SCIENCE*, 2008, vol. 43 (20), 6630-6641
- **CROLL S. G.** Application and limitations of current understanding to model failure modes in coatings. *JOURNAL OF COATINGS TECHNOLOGY AND RESEARCH*, 2012, vol. 10 (1), 15-27
- **CROLL S. G. et al.** Statistical approaches for predicting weathering degradation and service life. *PROCESS IN ORGANIC COATINGS*, 2006, vol. 55 (2), 75-87
- **GUSEVA O. et al.** Service life prediction for aircraft coatings. *POLYMER DEGRADATION AND STABILITY*, 2003, vol. 82 (1), 1-13
- **KIIL S.** Model-based analysis of photoinitiated coating degradation under artificial exposure conditions. *JOURNAL OF COATINGS TECHNOLOGY AND RESEARCH*, 2012, vol. 9 (4), 375-398